# EUROPÄISCHE PATENTANMELDUNG

(11) **EP 4 467 130 A1**
(43) Veröffentlichungstag der Anmeldung: **27.11.2024**
(21) Anmeldenummer: 24172414.5
(22) Anmeldetag: 25.04.2024
(51) Int. Cl.: A61K 9/46, A61K 36/185, A61K 36/45, A61K 45/06, A61P 13/10, A23L 33/105

(54) **DARREICHUNGSFORM AUF BASIS VON PFLANZENEXTRAKTEN**

(30) Priorität: 26.05.2023 DE 102023113947; 29.06.2023 DE 102023117188
(71) Anmelder: Maria Clementine Martin Klosterfrau Vertriebsgesellschaft mbH, 50670 Köln (DE)
(72) Erfinder: OBERBÖRSCH, Stefan, Dr., 50670 Köln (DE)
(74) Vertreter: Von Rohr Patentanwälte Partnerschaft mbB

(57) **Zusammenfassung**

Die vorliegende Erfindung betrifft das Gebiet der Medizin sowie der Nahrungsergänzungsmittel, insbesondere das technische (d. h. medizinischpharmazeutische bzw. nahrungsmitteltechnische) Gebiet der prophylaktischen und/oder therapeutischen Behandlung von Infektionen und/oder entzündlichen Erkrankungen des Urogenitaltraktes bzw. die unterstützend präventive und/oder kurative Sicherstellung bzw. Förderung der Blasengesundheit. Insbesondere betrifft die vorliegende Erfindung eine Darreichungsform, insbesondere für die orale Applikation, vorzugsweise zur Verwendung bei der prophylaktischen und/oder therapeutischen Behandlung von Infektionen und/oder entzündlichen Erkrankungen des Urogenitaltraktes, insbesondere der Harnwege, und/oder zur unterstützenden präventiven und/oder kurativen Sicherstellung und/oder Förderung der Blasengesundheit.

## Beschreibung

Die vorliegende Erfindung betrifft das Gebiet der Medizin sowie der Nahrungsergänzungsmittel, insbesondere das technische (d. h. medizinischpharmazeutische bzw. nahrungsmitteltechnische) Gebiet der prophylaktischen und/oder therapeutischen Behandlung von Infektionen und/oder entzündlichen Erkrankungen des Urogenitaltraktes bzw. die unterstützend präventive und/oder kurative Sicherstellung bzw. Förderung der Blasengesundheit.

Insbesondere betrifft die vorliegende Erfindung eine Darreichungsform, insbesondere pharmazeutische Darreichungsform, insbesondere für die orale Applikation, vorzugsweise zur Verwendung bei der prophylaktischen und/oder therapeutischen Behandlung von Infektionen und/oder entzündlichen Erkrankungen des Urogenitaltraktes, insbesondere der Harnwege, und/oder zur unterstützenden präventiven und/oder kurativen Sicherstellung und/oder Förderung der Blasengesundheit.

Darüber hinaus betrifft die vorliegende Erfindung auch die Verwendung einer Darreichungsform nach der vorliegenden Erfindung im Nahrungsergänzungsbereich, insbesondere zur Vorbeugung von Infektionen und/oder entzündlichen Erkrankungen des Urogenitaltraktes, insbesondere der Harnwege, und/oder bei der, insbesondere ergänzenden, präventiven und/oder kurativen Versorgung mit Nährstoffen und aktiven Inhaltsstoffen zur Sicherstellung und/oder Förderung der Blasengesundheit, sowie eine Brausezubereitung, insbesondere für die orale Applikation, insbesondere in Form eines losen oder gepressten Pulvers, und außerdem deren Verwendung sowie eine Brausetablette, und eine Verpackungseinheit, enthaltend mindestens eine Darreichungsform, Brausezubereitung oder Brausetablette nach der vorliegenden Erfindung.

Infektionen bzw. entzündliche Erkrankungen des Urogenitaltraktes, welche insbesondere die Harnwege betreffen, werden synonym auch als Harnwegsinfekte bezeichnet. Hierunter versteht man eine durch Krankheitserreger verursachte Infektion der ableitenden Harnwege. Dabei kann eine Unterteilung im Hinblick auf die von der Infektion betroffenen Organe bzw. Strukturen in einen sogenannten unteren Harnwegsinfekt und einen oberen Harnwegsinfekt erfolgen, wobei der untere Harnwegsinfekt im Wesentlichen nur die Harnröhre und die Harnblase betrifft und der obere Harnwegsinfekt eine Beteiligung der Nieren bzw. Harnleiter mit umfasst.

Der untere Harnwegsinfekt stellt meistens das Primärstadium der Infektionskrankheit dar, welches bei entsprechender Schwere der Erkrankung bzw. bei erfolgloser Behandlung zu einer Ausbreitung im Rahmen eines oberen Harnwegsinfektes führen kann. Sofern die Harnblase von dem Infekt betroffen ist, spricht man diesbezüglich von einer, insbesondere bakteriell hervorgerufenen, Zystitis.

In den meisten Fällen kommt ein Harnwegsinfekt über den Aufstieg von Erregern über die Harnröhre zustande, was auch als aszendierende Infektion bezeichnet wird.

Als die Harnwegsinfektion verursachende Erreger kommen insbesondere gramnegative Bakterien bzw. Erreger in Betracht, wobei *Escherichia coli* mit 80 % der Fälle als häufigster Erreger von Harnwegsinfekten auftritt. Jedoch können Harnwegsinfekte auch durch grampositive Kokken, Mykoplasmen, Ureaplasmen, Hefen, Chlamydien, Viren oder dergleichen hervorgerufen werden.

Im Allgemeinen stammen die Erreger von Infekten der Harnwege oftmals aus der körpereigenen Darmflora, wobei die Infektion häufig dadurch entsteht, dass diese Erreger zur äußeren Harnröhrenöffnung gelangen und von dort die Harnröhre hinauf in die Harnblase wandern, wo sie beispielsweise zu der zuvor beschriebenen Zystitis führen können. Bei einem weiteren Aufstieg kann es zu einer Nierenbeckenentzündung, einschließlich der Beteiligung des Nierengewebes selbst (Pyelonephritis) und schließlich zu einer äußerst gefährlichen Blutvergiftung (Urosepsis) kommen. Aufgrund der anatomischen Voraussetzungen sind Frauen von Harnwegsinfekten häufiger betroffen als Männer.

Eine wichtige Rolle im Verlauf der Infektion spielt die Kontaktaufnahme und Adhäsion der Erreger mit dem Zielgewebe, wie beispielsweise dem Epithel der Harnwege, wobei bestimmte Oberflächenmoleküle der Erreger diesbezüglich von hoher Bedeutung sind.

Die mit der Harnwegsinfektion einhergehenden Komplikationen sind umfangreich und mitunter schwerwiegend: So führt beispielsweise eine Infektion der Harnröhre selbst zu Schmerzen beim Urinieren oder löst auch einen unangenehmen Juckreiz aus. Zudem ist der Harzfluss oftmals vermindert. Charakteristisch ist auch ein häufiger Harndrang, bei dem die betroffenen Personen aber nur kleine Mengen an Urin absetzen können. Mitunter können auch blutige Beimengungen im Urin vorkommen. Ist bei Männern die Prostata mitbetroffen, zeigen die betroffenen Personen oftmals ein schweres Krankheitsbild mit Schmerzen im Unterbauch, in der Darmregion und Fieber. Gleichermaßen ist es auch möglich, dass die Erkrankung nahezu ohne Symptome verläuft oder dass allenfalls uncharakteristische Symptome auftreten. Bei rund 30 % der Blasenentzündungen zeigt sich beispielsweise eine symptomlose Nierenbeckenentzündung. Aufgrund der zum Teil nur geringen Spezifität bzw. Ausprägung der Symptome ist die Diagnose von Harnwegsinfekten nicht immer einfach bzw. eindeutig.

Eine weitere Problematik bei entzündlichen Erkrankungen der Harnwege liegt darin, dass häufig Rezidive auftreten, wobei in diesem Zusammenhang auch von chronisch rezidivierenden Harnwegsinfekten gesprochen wird. So tritt beispielsweise bei 50 % der weiblichen Patienten ein Rezidiv des Infektes auf. Tritt der Infekt binnen vierzehn Tagen nach dem Primärinfekt auf, ist von einem Überleben des Erregers im Urogenitaltrakt trotz vorübergehender klinischer Besserung auszugehen. Tritt ein Harnwegsinfekt mehr als vierzehn Tage nach klinischer Abheilung des ersten Infektes auf, ist dagegen von einer Neuinfektion auszugehen. Chronisch rezidivierende Harnwegsinfekte stellen dabei insbesondere aufgrund der damit einhergehenden teilweise schmerzhaften Symptomatik eine hohe Belastung für die betroffenen Personen dar.

Zur Behandlung von Harnwegsinfekten wird im Allgemeinen in erster Linie darauf abgestellt, die entsprechenden Erreger bzw. Krankheitskeime zu eliminieren, wobei beispielsweise antibiotische Therapieansätze im Stand der Technik in Betracht gezogen werden. Nachteilig sind diesbezüglich jedoch die mit der Verabreichung von Antibiotika einhergehenden Komplikationen und Nebenwirkungen, wie beispielsweise allergische Reaktionen, Hypoglykämien und durch die Antibiotika induzierte Hautschädigungen. Zudem können auch Resistenzbildungen auftreten.

Im Stand der Technik sind zur Behandlung von Harnwegsinfekten auch pflanzliche Präparate bekannt. So werden neben bestimmten Teezubereitungen und dergleichen im Stand der Technik pflanzliche Präparate aus Moosbeere bzw. Kranbeere ("Cranberry") in Form von Monopräparaten eingesetzt, wobei der diesbezügliche Therapieerfolg jedoch nicht immer überzeugend ist. Auch kommen im Rahmen der Behandlung von Harnwegsinfekten unterstützende Allgemeinmaßnahmen hinzu, wie eine Erhöhung der Trinkmenge, wodurch aufgrund der erhöhten Urinproduktion die Krankheitserreger gewissermaßen aus den besiedelten Gebieten ausgeschwemmt werden sollen. Dieser Ansatz kann jedoch allenfalls begleitend zu einer Primärtherapie in Betracht kommen. Insgesamt führen die im Stand der Technik vorgesehenen medikamentösen Ansätze zur Vorbeugung bzw. Behandlung von Harnwegsinfekten nicht immer zu einem ausreichenden Therapieerfolg und weisen oftmals auch gravierende Nebenwirkungen auf.

Vor diesem Hintergrund besteht - insbesondere auch aufgrund der großen Verbreitung und des häufig rezidivierenden Auftretens von Harnwegsinfektionen - ein großer Bedarf, geeignete Zusammensetzungen und Darreichungsformen sowie Therapieformen zur Behandlung von Harnwegsinfekten bereitzustellen.

Insgesamt besteht im Stand der Technik also weiterhin ein Bedarf an der Bereitstellung eines effizienten Konzepts zur prophylaktischen bzw. therapeutischen Behandlung von insbesondere rezidivierenden Infektionen und/oder entzündlichen Erkrankungen des Urogenitaltraktes und/oder eines effizienten Konzepts zur unterstützenden präventiven und/oder kurativen Sicherstellung bzw. Förderung der Blasengesundheit, insbesondere wobei das Konzept eine effiziente bzw. vergleichsweise hohe Wirksamkeit mit einer guten bzw. insbesondere im Alltag leichte handhabbaren Anwendbarkeit kombiniert.

Vor dem Hintergrund des zuvor geschilderten Standes der Technik liegt der vorliegenden Erfindung somit die Aufgabe zugrunde, ein effizientes Konzept bzw. eine diesbezügliche Darreichungsform bereitzustellen, welche(s) eine effiziente Behandlung von insbesondere rezidivierenden Infektionen und/oder entzündlichen Erkrankungen des Urogenitaltraktes und/oder eine präventive und/oder kurative Sicherstellung bzw. Förderung der Blasengesundheit ermöglicht, wobei in diesem Zusammenhang die zuvor angeführten Nachteile des Standes der Technik überkommen oder zumindest abgeschwächt werden sollen.

Insbesondere liegt der vorliegenden Erfindung die Aufgabe zugrunde, entsprechende Darreichungsformen bereitzustellen, welche im Rahmen der Behandlung von insbesondere rezidivierenden Infektionen und/oder entzündlichen Erkrankungen des Urogenitaltraktes und/oder bei der unterstützenden präventiven und/oder kurativen Sicherstellung bzw. Förderung der Blasengesundheit eine hervorragende Wirkeffizienz sowie eine unkomplizierte Applikation bei gleichzeitig guter Verträglichkeit sowie auch einer angenehmen sensorischen Empfindung aufweisen.

In vollkommen überraschender Weise hat die Anmelderin nunmehr gefunden, dass sich eine sehr spezielle Darreichungsform, insbesondere zur oralen Applikation, in hervorragender Weise für die prophylaktische bzw. therapeutische Behandlung von insbesondere rezidivierenden Infektionen und/oder entzündlichen Erkrankungen des Urogenitaltraktes und/oder als effizientes Konzept zur unterstützenden präventiven und/oder kurativen Sicherstellung bzw. Förderung der Blasengesundheit eignet, und zwar insbesondere im Hinblick auf die präventive Sicherstellung bzw. kurative Förderung einer normalen Funktion des Urogenitaltraktes, insbesondere der Harnblase, sowie gleichermaßen im Hinblick auf die prophylaktischen und/oder therapeutische Behandlung von Entzündungen des Urogenitaltraktes, was jeweils auf Grundlage der vorteilhaften Wirkstoffkombination in der erfindungsgemäßen Darreichungsform erreicht wird, die sich damit sowohl für den Einsatz als Nahrungsergänzungsmittel als auch als Medikament, zumindest zur Linderung des Verlaufs von Urogenitaltraktinfektionen, vorteilhaft anbietet.

Zur Lösung der zuvor geschilderten Problemstellung schlägt die vorliegende Erfindung somit - gemäß einem **ersten** Aspekt der vorliegenden Erfindung - eine Darreichungsform, insbesondere eine Nahrungsergänzungsmittel- und/oder pharmazeutische Darreichungsform, insbesondere für die orale Applikation, vorzugsweise zur Verwendung bei der prophylaktischen und/oder therapeutischen Behandlung von Infektionen und/oder entzündlichen Erkrankungen des Urogenitaltraktes, insbesondere der Harnwege, und/oder zur unterstützenden präventiven und/oder kurativen Sicherstellung und/oder Förderung der Blasengesundheit, gemäß Anspruch 1, vor. Jeweils vorteilhafte Weiterbildungen und Ausgestaltungen dieses Erfindungsaspekts sind Gegenstand der diesbezüglichen Neben- bzw. Unteransprüche.

Weiterer Gegenstand der vorliegenden Erfindung - gemäß einem **zweiten** Aspekt der vorliegenden Erfindung - ist zudem eine Darreichungsform nach der vorliegenden Erfindung zur Verwendung bei der prophylaktischen und/oder therapeutischen Behandlung von Infektionen und/oder entzündlichen Erkrankungen des Urogenitaltraktes, insbesondere der Harnwege, nach Anspruch 15.

Wiederum weiterer Gegenstand der vorliegenden Erfindung - gemäß einem **dritten** Aspekt der vorliegenden Erfindung - ist die Verwendung einer Darreichungsform nach der vorliegenden Erfindung im Nahrungsergänzungsbereich, insbesondere zur nicht-therapeutischen und/oder nicht-medizinischen Vorbeugung von Infektionen und/oder entzündlichen Erkrankungen des Urogenitaltraktes nach Anspruch 16.

Ebenfalls Gegenstand der vorliegenden Erfindung - gemäß einem **vierten** und **fünften** Aspekt der vorliegenden Erfindung - ist zudem eine Brausezubereitung nach Anspruch 17, insbesondere für die orale Applikation, insbesondere in Form eines losen oder gepressten Pulvers, vorzugsweise eine Brausetablette, bestehend aus einer Darreichungsform nach der vorliegenden Erfindung zur Verwendung bei der prophylaktischen und/oder therapeutischen Behandlung von Infektionen und/oder entzündlichen Erkrankungen des Urogenitaltraktes, insbesondere der Harnwege, und/oder zur unterstützenden präventiven und/oder kurativen Sicherstellung und/oder Förderung der Blasengesundheit, und die Verwendung einer Brausezubereitung nach der vorliegenden Erfindung im Nahrungsergänzungsbereich, insbesondere zur nicht-therapeutischen und/oder nicht-medizinischen Vorbeugung von Infektionen und/oder entzündlichen Erkrankungen des Urogenitaltraktes, insbesondere der Harnwege, nach Anspruch 18.

Wiederum weiterer Gegenstand der vorliegenden Erfindung - gemäß einem **sechsten** Aspekt der vorliegenden Erfindung - ist eine Brausetablette (1), insbesondere umfassend eine Nahrungsergänzungsmittel- und/oder pharmazeutische Darreichungsform, nach Anspruch 19.

Schließlich Gegenstand der vorliegenden Erfindung - gemäß einem **siebten** Aspekt der vorliegenden Erfindung - ist eine Verpackungseinheit nach Anspruch 20.

Es versteht sich von selbst, dass Ausgestaltungen, Ausführungsformen, Vorteile und dergleichen, welche nachfolgend zu Zwecken der Vermeidung von Wiederholungen nur zu einem Erfindungsaspekt aufgeführt sind, selbstverständlich auch in Bezug auf die übrigen Erfindungsaspekte entsprechend gelten, ohne dass dies einer gesonderten Erwähnung bedarf.

Weiterhin versteht es sich von selbst, dass bei nachfolgenden Angaben von Werten, Zahlen und Bereichen die diesbezüglichen Werte-, Zahlen- und Bereichsangaben nicht beschränkend zu verstehen sind; es versteht sich für den Fachmann von selbst, dass einzelfallbedingt oder anwendungsbezogen von dem angegebenen Bereich bzw. Angaben abgewichen werden kann, ohne dass der Rahmen der vorliegenden Erfindung verlassen ist.

Zudem gilt, dass alle im Folgenden genannten Werte- bzw. Parameterangaben oder dergleichen grundsätzlich mit genormten bzw. standardisierten oder explizit angegebenen Bestimmungsverfahren oder aber mit dem Fachmann auf diesem Gebiet an sich geläufigen Bestimmungsmethoden ermittelt bzw. bestimmt werden können.

Ferner ist bei allen nachstehend genannten relativen bzw. prozentualen, insbesondere gewichtsbezogenen Mengenangaben zu beachten, dass diese Angaben im Hinblick auf das herangezogene Bezugssystem (z. B. Darreichungsform) vom Fachmann derart auszuwählen bzw. zu kombinieren sind, dass in der Summe - gegebenenfalls unter Einbeziehung weiterer Komponenten bzw. Inhaltsstoffe bzw. Zusatzstoffe bzw. Bestandteile, insbesondere wie nachfolgend definiert - stets 100 % bzw. 100 Gew.-% resultieren. Dies versteht sich für den Fachmann aber von selbst.

Darüber hinaus gilt für die Beschreibung der vorliegenden Erfindung, dass die jeweils im Zusammenhang mit den speziellen Ausgestaltungen, Ausführungsformen, Vorteilen, Beispielen oder dergleichen angeführten Merkmale der vorliegenden Erfindung auch in deren Kombination als offenbart gelten. Somit gelten vorliegend auch übergeordnete Kombinationen einzelner oder mehrerer Merkmale, welche für jeweilige Ausgestaltungen, Ausführungsformen, Anwendungsbeispiele oder dergleichen angeführt sind, als offenbart.

Insbesondere gilt für die die Erfindung charakterisierenden Merkmale zudem, dass auch beliebige Kombinationen dieser Merkmale als offenbart gelten, wobei Ausführungsformen gleicher Präferenz der verschiedenen Merkmale in ihrer Kombination bevorzugt sind (z.B. Mengen bzw. Mengenbereiche der betreffenden Wirk- und Inhaltsstoffe gleicher Präferenz).

Weiterhin gilt in diesem Zusammenhang insbesondere auch, dass in Bezug auf die nachfolgend angeführten, die verschiedenen Inhaltsstoffe, insbesondere Wirkstoffe, der erfindungsgemäßen Darreichungsform betreffenden Mengenangaben, insbesondere relativen Mengenangaben oder absoluten Mengenangaben, jeweilige und die verschiedenen Inhaltsstoffe, insbesondere Wirkstoffe, betreffende Kombinationen gleicher Präferenz bzw. gleicher Bevorzugungsebene mit gleicher Präferenz bzw. Bevorzugung mitoffenbart sind. Ebenfalls sind auch sämtliche anderweitige Kombinationen (d.h. Kombinationen auf Basis unterschiedlicher Präferenzen bzw. unterschiedlicher Bevorzugungsebenen) mitoffenbart.

Dies vorausgeschickt, wird die vorliegende Erfindung nunmehr nachfolgend im Detail erläutert:
Gegenstand der vorliegenden Erfindung - gemäß einem **ersten** Aspekt der vorliegenden Erfindung - ist eine Darreichungsform, insbesondere eine Nahrungsergänzungsmittel- und/oder pharmazeutische Darreichungsform, insbesondere für die orale Applikation, vorzugsweise zur Verwendung bei der prophylaktischen und/oder therapeutischen Behandlung von Infektionen und/oder entzündlichen Erkrankungen des Urogenitaltraktes, insbesondere der Harnwege, und/oder zur unterstützenden präventiven und/oder kurativen Sicherstellung und/oder Förderung der Blasengesundheit,
wobei die Darreichungsform als Brausezubereitung, insbesondere in Form eines losen oder gepressten Pulvers, vorzugsweise in Form einer Brausetablette, ausgebildet ist,
wobei die Darreichungsform eine Kombination von mindestens einer Proanthocyanidine-enthaltenden Wirkstoffkomponente einerseits und mindestens einer diuretisch-wirksamen und/oder entzündungshemmenden Wirkstoffkomponente andererseits umfasst, und
wobei die Kombination
   a) mindestens einen Extrakt, insbesondere mindestens zwei Extrakte, von Pflanzen aus der Familie der Heidekrautgewächse (Ericaceae), insbesondere der Gattung Heidelbeere (Vaccinium), einerseits und
   b) mindestens einen Extrakt von Pflanzen aus der Familie der Brennnesselgewächse (Urticaceae) andererseits
   enthält.

Im Rahmen der vorliegenden Erfindung ist es auf Grundlage der erfindungsgemäßen Darreichungsform mit der speziellen Wirkstoffkombination aus mindestens einem Extrakt von Pflanzen aus der Familie der Heidekrautgewächse (Ericaceae), insbesondere der Gattung Heidelbeere (Vaccinium), und mindestens einem Extrakt von Pflanzen aus der Familie der Brennnesselgewächse (Urticaceae), sowie zudem insbesondere auch in Kombination mit der Darreichung als Brausezubereitung, in völlig überraschender Weise gelungen, eine hocheffiziente sowie -effektive Wirkstoffkombination bzw. Kombination physiologisch aktiver Inhaltsstoffe in einer Darreichungsform bereitzustellen, welche ganz überwiegend auf natürlichen Inhaltsstoffen pflanzlichen Ursprungs beruht und nachweislich vorteilhaft dazu beiträgt, die prophylaktische bzw. therapeutische Behandlung von Infektionen des Urogenitaltraktes zu unterstützen sowie, für die Anwendung als Nahrungsergänzungsmittel, eine normale Funktion des Urogenitaltraktes zu fördern bzw. sicherzustellen, d.h. auf natürliche Weise schonend und gleichsam ernährungsphysiologisch effektiv die Blasengesundheit insgesamt zu schützen, ggf. wiederherzustellen und nicht zuletzt zu fördern.

In der erfindungsgemäßen Darreichungsform werden vorteilhaft vor allem physiologisch wertvolle Inhaltsstoffe bzw. hocheffektive Wirkstoffe pflanzlichen Ursprungs verwendet, so dass mittels der erfindungsgemäßen Darreichungsform auf weitestgehend natürliche und - insbesondere mit unterschiedlichsten Ernährungsgewohnheiten - gut verträgliche Weise ein Beitrag zur Förderung bzw. Wahrung der Blasengesundheit bzw. zur präventiven sowie auch therapeutischen Behandlung von Entzündungen bzw. Erkrankungen des Urogenitaltraktes (z.B. Zystitis etc.) geleistet werden kann.

Die vorteilhafte, die Blasengesundheit unterstützende, Wirkweise erfindungsgemäßer Darreichungsformen begründet sich insbesondere darin, dass Extrakte von Pflanzen aus der Familie der Heidekrautgewächse, die unter anderem hohe Anteile an Tanninen, Proanthocyanidinen, Anthocyanidinen, Flavonoiden, Benzoesäure, Vitamin C, Natrium, Phosphor und Kalium enthalten, in Kombination mit Extrakten von Pflanzen aus der Familie der Brennnesselgewächse, die sich insbesondere durch diuretisch-wirksame sowie entzündungshemmende Eigenschaften auszeichnen, eingesetzt werden. Insbesondere die in den Heidekrautgewächsen enthaltenen Tannine, speziell Proanthocyanidine, scheinen dabei - ohne sich auf diese Theorie festlegen zu wollen - maßgeblich für die therapeutische Wirkung der erfindungsgemäßen Darreichungsform im Zusammenhang mit Urogenitaltraktinfektionen bzw. die vorteilhaften nahrungsergänzenden Eigenschaften in Bezug auf die Blasengesundheit zu sein. Die Tannine und insbesondere Proanthocyanidine der Heidekrautgewächsextrakte können insbesondere das Anhaften von Bakterien, insbesondere von *E. coli*, an der Blasenschleimhaut, insbesondere mittels der P-Fimbrien, an den uroepithelialen Zellen der Blasenwand, hemmen bzw. verhindern, wodurch die Ausbreitung der Keime in der Blase insgesamt stark eingeschränkt bzw. minimiert werden kann.

Auf diese Weise kann sowohl erreicht werden, dass Blaseninfekte schneller ausheilen, als auch, dass diesen effizient vorgebeugt wird, indem bereits das initiale Anhaften von Keimen an der Blasenschleimhaut unterbunden, zumindest aber deutlich erschwert, werden kann. Dementsprechend dient die erfindungsgemäße Darreichungsform vorteilhaft einerseits sowohl der prophylaktischen als auch der therapeutischen Behandlung von Erkrankungen bzw. Entzündungen des Urogenitaltraktes. Andererseits kann die erfindungsgemäße Darreichungsform insbesondere auch in Form eines Nahrungsergänzungsmittels eingesetzt werden und als solches ernährungsphysiologisch einen bedeutsamen Beitrag zum Erhalt bzw. der Förderung der Blasengesundheit leisten. Insbesondere die vorteilhafte Anwendung als Nahrungsergänzungsmittel, das regelmäßig, bspw. täglich, eingenommen wird, kann dabei dauerhaft zu einer Steigerung des Komforts beim Wasserlassen sowie zu einer anhaltend normalen Funktion des Urogenitaltraktes wirkungsvoll beitragen.

Vorteilhaft zählen dabei insbesondere die Extrakte von Preiselbeere (*Vaccinium vitis-idaea* L.) und Großfrüchtiger Moosbeere (*Vaccinium macrocarpon Aiton*, auch bekannt als Kranbeere bzw. Cranberry) innerhalb der Gruppe der Pflanzenextrakte aus der Familie der Heidekrautgewächsen zu den beliebtesten sowie auch wirkungsvollsten Optionen für eine effektiven Prophylaxe in Bezug auf Harnwegsinfekte bzw. für eine wirkungsvolle ergänzende Nährstoffversorgung zur Sicherstellung bzw. Förderung der Blasengesundheit. In den beiden vorgenannten sowie im Rahmen der Erfindung bevorzugten Pflanzenextrakten können vergleichsweise hohe Mengen an Tanninen, insbesondere Proanthocyanidinen, gefunden werden, sodass Pflanzenextrakte von Preiselbeere und Moosbeere, insbesondere von Preiselbeere, die Adhäsion von Bakterien an der Blasenwand vergleichsweise besonders effizient unterbinden können. Darüber hinaus enthalten sowohl Preiselbeer- als auch Moosbeeren-Extrakte hohe Mengen an weiteren, physiologisch als vorteilhaft zu bewertenden sekundären Pflanzenstoffen sowie auch Mineralstoffen und Vitaminen. Auf dieser Grundlage können die im Rahmen der Erfindung eingesetzten Pflanzenextrakte, insbesondere von Preiselbeere und Großfrüchtiger Moosbeere, vorteilhaft auch zum allgemeinen Wohlbefinden sowie zu einer ausgewogenen Versorgung des Körpers mit essenziellen bzw. relevanten Nährstoffen, Mineralstoffen und Vitaminen beitragen.

Darüber hinaus vorteilhaft umfasst die erfindungsgemäße Darreichungsform ebenfalls Extrakte von Pflanzen aus der Familie der Brennnesselgewächse, die insbesondere diuretisch-wirksame sowie auch entzündungshemmende Wirkstoffkomponenten umfassen. Durch die Verwendung von Pflanzenextrakten aus Brennnesselgewächsen, insbesondere von Extrakten aus Brennnesselblättern (*Urticae folium*), kann so auf natürliche sowie vor allem auch schonende Weise der Harnfluss mild angetrieben und eine intensivierte Spülung der Blase erreicht werden. Gleichzeitig tragen die entzündungshemmenden Wirkkomponenten in dem Extrakt effektiv zu einer Reduktion eines möglichen Infektionsrisikos bzw. einer Milderung bestehender Infektionen bei.

Die diuretische Wirkung von Extrakten von Pflanzen aus der Familie der Brennnesselgewächse beruht dabei vorteilhaft insbesondere auf einem hohen Kaliumgehalt im Zusammenspiel mit weiteren Wirkstoffen, wie insbesondere den diuretisch wirksamen Extraktbestandteilen Kaffeoyläpfelsäure und Chlorogensäure. Die genannten Säuren wirken gleichsam auch entzündungshemmend, was insgesamt als vorteilhaft für den Aspekt der Förderung bzw. des Erhalts der Blasengesundheit zu bewerten ist, indem schonend und mild ein verbessertes Durchspülen der Harnwege sowie gleichzeitig ein entzündungshemmendes bzw. -milderndes Milieu erzielt werden kann. Darüber hinaus zeichnen sich Extrakte von Pflanzen aus der Familie der Brennnesselgewächse auch durch hohe Gehalte weiterer als physiologisch wertvoll bewerteter Inhaltsstoffe wie beispielsweise Chlorophyll, Carotinoiden, Lutein und Mineralstoffen, wie beispielsweise Kieselsäure, Kalium, Calcium und Eisen, aus.

Mit der erfindungsgemäßen Darreichungsform wird also vorteilhaft eine besonders wirkungsvolle Wirkstoff- bzw. Nährstoffkombination im Hinblick auf die Behandlung von Infektionen oder Entzündungen des Urogenitaltraktes bzw. die Förderung bzw. den Erhalt der Blasengesundheit bereitgestellt. Die Kombination aus insbesondere diuretisch-wirksamen und entzündungshemmenden Wirkstoffkomponenten aus Extrakten von Pflanzen aus der Familie der Brennnesselgewächse und Proanthocyanidin-reichen Pflanzenextrakten aus der Familie der Heidekrautgewächse kann dabei letztlich sogar in synergistischer Weise ein wirkungsvolles sowie nachhaltiges Ausschwemmen von Bakterien aus der Blase erreichen, in dem der Harnfluss mild angetrieben und das Anhaften von Bakterien in der Blasenschleimhaut erschwert wird, wobei zusätzlich auch ein entzündungshemmendes Milieu geschaffen wird. Mit der erfindungsgemäßen Darreichungsform kann also erreicht werden, dass Bakterien, die in die Blase gelangen, einerseits schlechter in der Blase verweilen, insbesondere da ein Anhaften an die Blasenschleimhaut erschwert ist, und andererseits verstärkt aus der Blase ausgeschwemmt werden. Auf diese Weise wird besonders effizient sowie nachhaltig und anhaltend eine deutliche Reduktion der Bakterienbelastung in der Blase erreicht bzw. kann einem zu einer Entzündung führenden Bakterienbefall schonend und wirksam vorgebeugt werden. Damit kann vorteilhaft die Blasengesundheit erhalten bzw. gefördert werden und kann ein wirkungsvoller Beitrag zur prophylaktischen und/oder therapeutischen Behandlung von Infektionen des Urogenitaltraktes geleistet werden.

Die vorteilhaften Wirkungen der Extrakte von Pflanzen aus der Familie der Heidekrautgewächse bzw. von Pflanzen aus der Familie der Brennnesselgewächse werden in der erfindungsgemäßen Darreichungsform weiterhin vorteilhaft dadurch komplementiert, dass die Darreichungsform als Brausezubereitung ausgebildet sein kann. Die Darreichungsform kann dann im Rahmen der Applikation vorteilhaft in Wasser aufgelöst und als brausiges bzw. sprudelndes Getränk eingenommen werden, was wertvollerweise dazu beiträgt, dass zusätzliche Flüssigkeit aufgenommen wird, so dass wiederum vorteilhaft das Ausschwemmen von Keimen aus der Blase durch eine Erhöhung des Harnflusses unterstützt wird. Gleichermaßen kann ein Anhaften von Bakterien zusätzlich erschwert werden, indem durch den insgesamt erhöhten Hahnfluss die Interaktion mit der Blasenschleimhaut gestört und so insgesamt die Eliminierung von Bakterien aus der Blase nochmal deutlich begünstigt wird.

Darüber hinaus gestattet die bevorzugte Ausgestaltung der erfindungsgemäßen Darreichungsform als Brausezubereitung eine effiziente und möglichst zweckmäßige sowie konsumentenfreundliche Formulierung der Darreichungsform, in dem eine gute Dosierbarkeit möglich ist und Dosiereinheiten, insbesondere in Form von Brausetabletten, mit einem besonders vorteilhaften Verhältnis aus Menge und Volumen der Dosiereinheit zu Wirkstoffgehalt der Darreichungsform eingestellt werden kann. Weiterhin gestattet diese bevorzugte Ausgestaltung als Brausetablette vorteilhaft eine einfache Anwendbarkeit, insbesondere auch im Alltag als Nahrungsergänzungsmittel, indem durch einfaches Auflösen der Tablette ein anwendungsfertiges Getränk erhalten werden kann oder die Tablette auch ohne weiteres direkt oral appliziert werden kann.

Darüber hinaus können erfindungsgemäße Darreichungsformen vorteilhaft auch weitere physiologisch wertvolle Inhaltsstoffe enthalten, insbesondere beispielsweise weitere Vitamine und Mineralstoffe, wobei Vitamin C und Magnesium als Mineralstoff bevorzugt sind. Magnesium trägt dabei vorteilhaft zum Erhalt des Elektrolytgleichgewichts im Körper bei und zeichnet sich darüber hinaus auch durch eine muskelrelaxierende sowie krampflösende Wirkung aus, was insbesondere auch bei mit Infektionen des Urogenitaltraktes, insbesondere der Harnblase, assoziierten Problemen im Zusammenhang mit der Urinabgabe hilfreich unterstützen kann. In höherer Dosierung kann Magnesium darüber hinaus entzündungshemmend wirken, so dass auf diese Weise ebenfalls zur therapeutischen bzw. prophylaktischen Behandlung von Infektionen und/oder Entzündungen des Urogenitaltraktes beigetragen werden kann.

Vitamin C trägt zudem vorteilhaft zu einer normalen Funktion des Immunsystems bei, was die Förderung bzw. den Erhalt der Blasengesundheit positiv beeinflussen und gleichermaßen auch im Rahmen der therapeutischen und/oder prophylaktischen Behandlung von Infektionen des Urogenitaltraktes vorteilhaft sein kann. Darüber hinaus kann Vitamin C dazu beitragen, die Eisenaufnahme in den Körper zu erhöhen, was insbesondere für Patienten bzw. Konsumenten, die unter einem Eisenmangel leiden bzw. von Natur aus zu einer verringerte Eisenaufnahme neigen, von Vorteil sein kann. Diese Wirkung von Vitamin C unterstützt somit insbesondere vorteilhaft auch die bessere Verwertung von Mineralstoffen, insbesondere Eisen, aus den in der erfindungsgemäßen Darreichungsform enthaltenen Pflanzenextrakten, so dass der Zusatz von Vitamin C letztlich zu einer besseren Verwertung der Nähr- und Wirkstoffe der Pflanzenextrakte führen kann.

Insgesamt wird mittels der erfindungsgemäßen Darreichungsform und auf Basis der speziellen Wirkstoffkombination von Extrakten von Pflanzen aus der Familie der Heidekrautgewächse und Extrakten von Pflanzen aus der Familie der Brennnesselgewächse, gegebenenfalls in Kombination mit weiteren Mineralstoffen und Vitaminen, wie insbesondere Magnesium und Vitamin C, und zudem vorzugsweise komplementiert durch die Formulierung der Darreichungsform als Brausezubereitung, die bevorzugt in Wasser aufgelöst als Getränk eingenommen werden kann, vorteilhaft erreicht, dass ein Konsument, der seine Blasengesundheit fördern möchte, bzw. Patient, der zu insbesondere rezidivierenden Harnwegsinfekten neigt, auf einfache und unkomplizierte Weise zum Trinken animiert und mit Inhalts- bzw. Wirkstoffen versorgt wird, die in der erfindungsgemäßen Kombination die Eliminierung von Bakterien aus der Blase begünstigen, indem ein Anhaften von Bakterien erschwert und ein intensiviertes Spülen bzw. Ausschwemmen aus der Blase gefördert wird. Der Hahnfluss wird also mild angetrieben und es wird ein Milieu, dass Infektionen erschwert bzw. unterbindet, geschaffen, sodass die Blase auf effiziente wie auch schonende Weise vor Bakterien geschützt und insgesamt gesund erhalten werden kann. Gleichsam kann einem Befall der Blase durch Bakterien effektiv ernährungsphysiologisch vorgebeugt werden bzw. kann auch eine akute Infektion mit der erfindungsgemäßen Darreichungsform behandelt werden, insbesondere mittels der in den verwendeten Pflanzenextrakten enthaltenen Wirkkomponenten. Schließlich kann die Darreichungsform auch einen wertvollen sowie positiven Beitrag zum Erhalt eines normalen sowie ausgewogenen Elektrolythaushalts leisten, und die Eisenaufnahmefähigkeit sowie die normale Funktion des Immunsystems fördern, wofür wiederum die bevorzugten und vorteilhaften Inhaltstoffe der erfindungsgemäßen Darreichungsform maßgeblich sind.

Der Begriff "Darreichungsform", wie er im Rahmen der vorliegenden Erfindung verwendet wird, ist dabei sehr breit zu verstehen und meint bzw. umfasst neben pharmazeutischen Präparaten bzw. Pharmazeutika und Arzneimittel als solchen auch sogenannte Medizinprodukte, Lebensmittel oder Nahrungsergänzungsmittel.

Der Begriff "Nahrungsergänzungsmittel", wie er im Rahmen der vorliegenden Erfindung verwendet wird, meint bzw. umfasst Lebensmittel-Produkte, die zur ergänzenden Versorgung des menschlichen Stoffwechsels mit bestimmten Nährstoffen wie Vitaminen oder Mineralstoffen gedacht sind. Nahrungsergänzungsmittel im Sinne der vorliegenden Erfindung enthalten diese Nährstoffe in konzentrierter und dosierter Form und werden insbesondere in lebensmitteluntypischer Form wie Tabletten, Trinkampullen oder Kapseln angeboten.

Der Begriff "Pharmazeutische Darreichungsform" meint bzw. umfasst eine Zubereitung aus Stoffen, die "zur Heilung oder zur Verhütung menschlicher Krankheiten" bestimmt sind oder sich zur Beeinflussung physiologischer Funktionen eignen oder eine medizinische Diagnose ermöglichen.

Darüber hinaus ist unter dem Begriff "orale Applikation" eine Verabreichung bzw. Einnahme der Darreichungsform über den Mund zu verstehen, so dass die Absorption der Wirk- bzw. aktiven Inhaltsstoffe der Darreichungsform bereits im Mund sowie auch im Magen beginnen kann.

Der Begriff "Brausezubereitung" meint bzw. umfasst eine insbesondere pulverförmige, vorzugsweise in Form einer Brausetablette ausgebildete, Zusammensetzung, die für die Herstellung sprudelnder Getränkezubereitungen geeignet ist bzw. auch direkt oral aufgenommen werden kann, wobei eine sprudelnde Empfindung unter Auflösung/Verflüssigung der Zubereitung im Mund hervorrufen wird.

Weiterhin meint bzw. umfasst der Begriff "Wirkstoffkomponente", wie dieser im Rahmen der vorliegenden Erfindung verwendet wird, insbesondere auf das Nahrungsergänzungsmittel bezogen "aktive Inhaltskomponente" bzw. auf das Pharmazeutikum bezogene die pharmazeutische Aktivkomponente.

Unter dem Begriff "Proanthocyanidine" ist im Rahmen der vorliegenden Erfindung ein Sammelbegriff für farblose, phenolische, der Gruppe der Tannine zugeordnete, Naturstoffe, die beim Erhitzen mit verdünnten Mineralsäuren eine Farbreaktion ergeben ("Proanthocyanidinreaktion") zu verstehen. Die Farbe entsteht durch die Bildung von Anthocyanidinen, den farbgebenden Komponenten der Anthocyane, die die rote, violette oder blaue Färbung von Blüten, Blättern und Früchten entsprechender Pflanzen bedingen.

Der Begriff "diuretisch-wirksam" meint bzw. umfasst, so wie er im Rahmen der vorliegenden Erfindung verwendet wird, die Wirkung einer vermehrten Ausschwemmung von Urin aus dem menschlichen Körper, welche durch eine verstärkte Harnerzeugung in den Nieren hervorgerufen wird.

Der Begriff "Extrakt" meint bzw. umfasst im Rahmen der Erfindung, insbesondere in Zusammenhang mit pharmazeutischen Zwecken, Zubereitungen, die mittels Extraktion ("Ausziehung") aus pharmazeutischen Drogen gewonnen werden, welche entweder direkt als Arzneimittel verwendet werden oder zu solchen weiterverarbeitet werden können, während im Nahrungsmittelbereich unter "Extrakt" ein Stoffgemisch verstanden wird, das durch selektive Anreicherung charakteristischer Bestandteile aus einem Ausgangsmaterial unter Verwendung von ggf. Extraktionslösemitttel/n gewonnen wird. Im Falle von Pflanzenextrakten stellen Pflanzen oder Teile davon in verarbeitetem oder unverarbeitetem Zustand das Ausgangsmaterial dar.

Nachfolgend wird die vorliegende Erfindung weiterführend und im Detail beschrieben, insbesondere im Hinblick auf weitere bevorzugte Ausgestaltungen bzw. bevorzugte Ausführungsformen.

Die vorliegende Erfindung betrifft dabei gemäß dem vorliegenden Aspekt auch eine erfindungsgemäße Darreichungsform, insbesondere Nahrungsergänzungsmittel- und/oder pharmazeutische Darreichungsform, insbesondere für die orale Applikation, vorzugsweise zur Verwendung bei der prophylaktischen und/oder therapeutischen Behandlung von Infektionen und/oder entzündlichen Erkrankungen des Urogenitaltraktes, insbesondere der Harnwege, und/oder zur unterstützenden präventiven und/oder kurativen Sicherstellung und/oder Förderung der Blasengesundheit, insbesondere wie zuvor beschrieben,
welche in Kombination und in jeweils wirksamen, insbesondere in jeweils Nahrungsergänzungsmittel- und/oder pharmazeutisch wirksamen, Mengen enthält:
a) mindestens eine Proanthocyanidine-enthaltende Wirkstoffkomponente in Form mindestens eines Extrakts, insbesondere mindestens zweier Extrakte, von Pflanzen aus der Familie der Heidekrautgewächse (Ericaceae), insbesondere der Gattung Heidelbeere (Vaccinium), und
b) mindestens eine diuretisch-wirksame und/oder entzündungshemmende Wirkstoffkomponente in Form mindestens eines Extrakts von Pflanzen aus der Familie der Brennnesselgewächse (Urticaceae).

Was nun die Auswahl des mindestens einen Extrakts, insbesondere der mindestens zwei Extrakte, von Pflanzen aus der Familie der Heidekrautgewächse (Ericaceae), insbesondere der Gattung Heidelbeere (Vaccinium), anbelangt, so kann dieser gewöhnlich aus einer Vielzahl von physiologisch verträglichen Extrakten von Pflanzen aus der Familie der Heidekrautgewächse (Ericaceae), insbesondere der Gattung Heidelbeere (Vaccinium), ausgewählt ein.

Im Rahmen der vorliegenden Erfindung hat es sich dabei als vorteilhaft erwiesen, wenn a) der mindestens eine Extrakt, insbesondere die mindestens zwei Extrakte, von Pflanzen aus der Familie der Heidekrautgewächse (Ericaceae), insbesondere der Gattung Heidelbeere (Vaccinium), ausgewählt ist/sind aus der Gruppe von Großfrüchtiger Moosbeere (*Vaccinium macrocarpon Aiton*), Kleinfrüchtiger Moosbeere (*Vaccinium microcarpum*), Gewöhnlicher Moosbeere (*Vaccinium oxycoccos* L.), Preiselbeere (*Vaccinium vitis-idaea* L.), Blaubeere (*Vaccinium myrtillus* L.), Amerikanischer Heidelbeere (*Vaccinium corymbosum* L.), Kanadischer Blaubeere (*Vaccinium myrtilloides* MICHX.) und/oder deren Mischungen, insbesondere Großfrüchtiger Moosbeere (*Vaccinium macrocarpon Aiton*), Preiselbeere (*Vaccinium vitis-idaea* L.) und/oder deren Mischungen.

Die vorgenannten Pflanzen aus der Familie der Heidekrautgewächse zeichnen sich insbesondere durch Wirk- bzw. Inhaltsstoffgehalte, insbesondere Proanthocyanidin-Gehalte, aus, die für die erfindungsgemäße Darreichungsform vorteilhaft und besonders geeignet sind, so dass die vorstehend beschriebenen vorteilhaften technischen Effekte und Wirkungen der Darreichungsform verlässlich erreicht werden können.

Weiter bevorzugt enthält die Darreichungsform in Kombination und in jeweils wirksamen, insbesondere in jeweils Nahrungsergänzungsmittel- und/oder pharmazeutisch wirksamen, Mengen mindestens zwei, insbesondere genau zwei, Extrakte aus Pflanzen der Familie der Heidekrautgewächse (Ericaceae), insbesondere der Gattung Heidelbeere (Vaccinium), wobei es sich als insbesondere vorteilhaft erwiesen hat, wenn a) einen ersten Extrakt a1) mindestens einen Extrakt von Preiselbeere (*Vaccinium vitis-idaea* L.) und einen zweiten Extrakt a2) mindestens einen Extrakt von Großfrüchtiger Moosbeere (*Vaccinium macrocarpon Aiton*) enthält, insbesondere hieraus besteht.

Die Extrakte von Preiselbeere (*Vaccinium vitis-idaea* L.) und Großfrüchtiger Moosbeere (*Vaccinium macrocarpon Aiton*) zeichnen sich durch besonders hohe Proanthocyanidin-Gehalte aus, insbesondere im Hinblick auf den Extrakt von Preiselbeere, so dass Darreichungsformen, die eine Kombination der beiden Pflanzenextrakte umfassen, besonders vorteilhafte Wirkungen in Bezug auf die prophylaktische und/oder therapeutische Behandlung von Infektionen und/oder entzündlichen Erkrankungen des Urogenitaltraktes, insbesondere der Harnwege, bzw. die unterstützende präventive und/oder kurative Sicherstellung und/oder Förderung der Blasengesundheit haben.

Bezüglich der Auswahl des Extrakts von Pflanzen aus der Familie der Brennnesselgewächse (Urticaceae) kann im Allgemeinen auf eine Vielzahl von Extrakten zurückgegriffen werden. Im Rahmen der vorliegenden Erfindung hat es sich für die Komponente b) bewährt, wenn der mindestens ein Extrakt von Pflanzen aus der Familie der Brennnesselgewächse (Urticaceae) ausgewählt ist aus der Gruppe von Großer Brennnessel (*Urtica dioica*), Kleiner Brennnessel (*Urtica urens*) und/oder deren Mischung ist.

Dabei ist es speziell bevorzugt, wenn b) einen Extrakt b1) mindestens einen Extrakt von Brennnesselblättern (*Urticae folium*) enthält, insbesondere hieraus besteht. Besonders die Verwendung von Brennnesselblättern hat sich im Rahmen der vorliegenden Erfindung als vorteilhaft ergeben, insbesondere da die diuretisch-wirksamen sowie entzündungshemmenden Wirk- bzw. Inhaltsstoffe der Brennnesselpflanze besonders in den Blättern enthalten sind bzw. in Extrakten aus diesen besonders gut angereichert werden können.

Mittels der erfindungsgemäß bevorzugten Extrakte von Pflanzen aus der Familie der Heidekrautgewächse sowie der Brennnesselgewächse werden die eingangs genannten Vorteile für die erfindungsgemäße Darreichungsform besonders wirkungsvoll realisiert, d.h. wenn im Rahmen der vorliegenden Erfindung Extrakte ausgewählt aus den vorgenannten Pflanzen aus der Familie der Heidekrautgewächse bzw. der Familie der Brennnesselgewächse verwendet werden, können erfindungsgemäße Darreichungsformen mit besonders vorteilhaften Wirkeigenschaften bzw. wertvollen nahrungsergänzenden Inhaltsstoffen erhalten werden.

Als besonders vorteilhaft hat es sich im Rahmen der Erfindung erwiesen, wenn die Darreichungsform in Kombination und in jeweils wirksamen, insbesondere in jeweils Nahrungsergänzungsmittel- und/oder pharmazeutisch wirksamen, Mengen enthält:
a1) mindestens einen Extrakt von Preiselbeere (*Vaccinium vitis-idaea* L.),
a2) mindestens einen Extrakt von Großfrüchtiger Moosbeere (*Vaccinium macrocarpon Aiton*) und
b1) mindestens einen Extrakt von Brennnesselblättern (*Urticae folium*);
   und/oder
wenn die Darreichungsform als Wirkstoffkomponente a) eine Kombination von a1) mindestens einem Extrakt von Preiselbeere (*Vaccinium vitis-idaea* L.) und a2) mindestens einem Extrakt von Großfrüchtiger Moosbeere (*Vaccinium macrocarpon Aiton*) und als Wirkstoffkomponente b) b1) mindestens einen Extrakt von Brennnesselblättern (*Urticae folium*) enthält.

Die Kombination von je mindestens einem Extrakt aus Preiselbeere, Großfrüchtiger Moosbeere und Brennnesselblättern liefert vorteilhaft eine Darreichungsform, die im Hinblick auf Ihre Wirkeigenschaften bzw. Eigenschaften als Nahrungsergänzungsmittel die eingangs genannten Vorteile in besonders ausgeprägter Weise verwirklicht. Insbesondere Extrakte von Preiselbeere sowie auch Großfrüchtiger Moosbeere zeigen im Zusammenhang mit der prophylaktischen und/oder therapeutischen Behandlung von Infektionen bzw. Entzündungen des Urogenitaltraktes aufgrund des jeweils hohen Proanthocyanidin-Gehalts eine hohe Wirksamkeit, insbesondere indem ein Anhaften von Bakterien erschwert bzw. gehemmt und dadurch insgesamt langfristig unterbunden werden kann.

In Kombination mit der vorteilhaften diuretischen sowie entzündungshemmenden Wirkung von Extrakten aus Brennnesselblättern kann durch den resultierenden milden Antrieb des Harnflusses ein erhöhtes Spülen der Blase erreicht und die Eliminierung von Bakterien damit begünstigt werden. Einer Ansammlung von Bakterien in der Blase kann so entgegengewirkt bzw. wirksam vorgebeugt werden. Die genannte Wirkstoffkombination trägt also als besonders geeigneter Weise zum Erhalt der Blasengesundheit bei bzw. gestattet eine besonders wirkungsvolle prophylaktische und/ oder therapeutische Behandlung von Blaseninfektionen.

Dementsprechend betrifft die vorliegende Erfindung gemäß dem vorliegenden Aspekt auch eine solche erfindungsgemäße Darreichungsform, insbesondere Nahrungsergänzungsmittel- und/oder pharmazeutische Darreichungsform, insbesondere für die orale Applikation, vorzugsweise zur Verwendung bei der prophylaktischen und/oder therapeutischen Behandlung von Infektionen und/oder entzündlichen Erkrankungen des Urogenitaltraktes, insbesondere der Harnwege, und/oder zur unterstützenden präventiven und/oder kurativen Sicherstellung und/oder Förderung der Blasengesundheit, insbesondere wie zuvor beschrieben,

die in Kombination und in jeweils wirksamen, insbesondere in jeweils Nahrungsergänzungsmittel- und/oder pharmazeutisch wirksamen, Mengen enthält:
a1) mindestens einen Extrakt von Preiselbeere (*Vaccinium vitis-idaea* L.),
a2) mindestens einen Extrakt von Großfrüchtiger Moosbeere (*Vaccinium macrocarpon Aiton*) und
b1) mindestens einen Extrakt von Brennnesselblättern (*Urticae folium).*

Für diese bevorzugte Ausführung der vorliegenden Erfindung hat es sich bewährt, wenn die Darreichungsform eine Kombination von mindestens einer Proanthocyanidine-enthaltenden Wirkstoffkomponente, insbesondere a1) und/oder a2), vorzugsweise a1), einerseits und mindestens einer diuretisch-wirksamen und/oder entzündungshemmenden Wirkstoffkomponente, insbesondere b1), andererseits umfasst.

Was nun die Formulierung bzw. Galenik der erfindungsgemäßen Darreichungsform anbelangt, so kann diese üblicherweise aus einer Vielzahl möglicher Formulierungen ausgewählt sein. Dabei hat es sich im Rahmen der vorliegenden Erfindung bewährt, wenn die Darreichungsform als Brausezubereitung, insbesondere in Form eines losen oder gepressten Pulvers, vorzugsweise in Form einer Brausetablette, ausgebildet ist.

Wie bereits eingangs erwähnt, ist die Zubereitung der erfindungsgemäßen Darreichungsform als Brausezubereitung, insbesondere in Form einer Brausetablette, mit einer Reihe von Vorteilen versehen. Die Darreichungsform kann insbesondere aufgelöst in Wasser als Getränk zubereitet und eingenommen werden, so dass auf unkomplizierte Weise eine zusätzliche Aufnahme von Flüssigkeit erreicht werden kann. Weiterhin ist eine schnelle Freisetzung sowie auch Resorption der Inhaltsstoffe der erfindungsgemäßen Darreichungsform möglich. Die erleichterte zusätzliche Flüssigkeitsaufnahme in Zusammenhang mit der besseren Verfügbarkeit der Inhaltsstoffe der Darreichungsform ist insbesondere im Rahmen der Behandlung von Infektionen der Blase bzw. einer diesbezüglichen Prophylaxe vorteilhaft, da der Hahnfluss mild angetrieben und eventuell in der Blase anhaftende Keime effizient ausgeschwemmt werden können, während gleichzeitig die Inhalts- bzw. Wirkstoffe der Darreichungsform schnell resorbiert und damit wirksam werden können.

Weiterhin gestattet die Darreichung als Brausezubereitung eine gute Portionier- bzw. Dosierung der Darreichungsform, sodass zu verabreichende bzw. einzunehmende Mengen der Darreichungsform gut abgemessen und kontrolliert werden können. Dies ist auch einer einfachen Applikation der erfindungsgemäßen Darreichungsform zuträglich und führt zu guten organoleptischen Eigenschaften ebenso wie einer guten Einnehmbarkeit der Darreichungsform auch für Kinder.

Darüber hinaus zur Zusammensetzung und Formulierung der erfindungsgemäßen Darreichungsform hat es sich im Rahmen der vorliegenden Erfindung, wenn die Extrakte jeweils zusammen mit und/oder in Kombination mit einem Träger formuliert sind und/oder vorliegen.

Diesbezüglich ist es bevorzugt, wenn der Träger ein wasserlöslicher oder wasserdispergierbarer, insbesondere Kohlenhydrat-basierter und/oder -haltiger, vorzugsweise Oligo- und/oder Polysaccharid-basierter und/oder -haltiger, Träger ist, bevorzugt Maltodextrin ist, und/oder wenn die Extrakte den Träger jeweils in Mengen in einem Bereich von 2 bis 70 Gew.-%, insbesondere 3 bis 60 Gew.-%, vorzugsweise 4 bis 55 Gew.-%, bevorzugt 5 bis 50 Gew.-%, bezogen auf die Gesamtzusammensetzung des Extrakts, enthalten.

Die Formulierung "Gesamtzusammensetzung" meint bzw. umfasst im Rahmen der vorliegenden Erfindung bezogen auf dem Extrakt dessen reinen Pflanzenextrakt-Anteil zusammen mit dem gegebenenfalls vorhandenen Träger sowie optional weitere Inhaltsstoffe des Extraktes.

Die Verwendung eines Trägers in bzw. mit den Pflanzenextrakten, die in erfindungsgemäßen Darreichungsformen eigesetzt werden sollen, bewirkt vorteilhaft eine bessere Verteilbarkeit der Extrakte in der Darreichungsform, indem eine homogenere und gleichmäßigere Verteilung erreicht werden kann. Es kann zudem auch eine stabilere Formulierung erhalten werden. Darüber hinaus gestattet die Verwendung eines Trägers vorteilhaft eine Verbesserung der Haltbarkeit der Darreichungsform, indem auf Basis des Trägers auch eine konservierende Wirkung bezüglich insbesondere der Wirk- bzw. Inhaltsstoffe der Extrakte erreicht werden kann. Durch diese Stabilisierung und Konservierung der Extrakte kann insgesamt auch eine verbesserte Lagerstabilität für die Darreichungsform erzielt werden.

Der vorzugsweise eingesetzte Träger weist vorteilhaft wasserlösliche bzw. wasserdispergierbare Löslichkeitseigenschaften auf, sodass die Darreichungsform bei Kontakt mit Wasser, insbesondere für die Formulierung als Brausezubereitung und entsprechend für die bevorzugte Darreichung als Brausegetränk, schnell aufgelöst und gut in Wasser verteilt werden kann. Die Verwendung des Trägers erleichtert dabei insbesondere die schnelle und rückstandsfreie Verteilung der Pflanzenextrakte in Wasser, indem der Träger bereits für die feste Darreichungsform sicherstellt, dass die Extrakte gleichmäßig und homogen in dieser verteilt vorliegen. In diesem Sinne trägt die Verwendung eines Trägers also vorteilhaft zu einer verbesserten Dispergierbarkeit der Pflanzenextrakte bei und verhindert das Verklumpen bzw. die Bildung von Klümpchen beim Lösen im Wasser. Insgesamt kann so auch eine Verbesserung insbesondere der organoleptischen Eigenschaften der Darreichungsform erzielt werden.

In diesem Sinne ist es im Rahmen der vorliegenden Erfindung daher bevorzugt, wenn a) der mindestens eine Extrakt, insbesondere die mindestens zwei Extrakte, von Pflanzen aus der Familie der Heidekrautgewächse (Ericaceae), insbesondere der Gattung Heidelbeere (Vaccinium), jeweils zusammen mit und/oder in Kombination mit einem Träger formuliert ist/sind und/oder vorliegt/vorliegen und/oder wenn b) der mindestens eine Extrakt von Pflanzen aus der Familie der Brennnesselgewächse (Urticaceae) jeweils zusammen mit und/oder in Kombination mit einem Träger formuliert ist und/oder vorliegt;
insbesondere wobei der Träger ein wasserlöslicher oder wasserdispergierbarer, insbesondere Kohlenhydrat-basierter und/oder -haltiger, vorzugsweise Oligo- und/oder Polysaccharid-basierter und/oder -haltiger, Träger ist, bevorzugt Maltodextrin ist, und/oder
insbesondere wobei der oder die Extrakte den Träger jeweils in Mengen in einem Bereich von 2 bis 70 Gew.-%, insbesondere 3 bis 60 Gew.-%, vorzugsweise 4 bis 55 Gew.-%, bevorzugt 5 bis 50 Gew.-%, bezogen auf die Gesamtzusammensetzung des Extrakts, enthält/enthalten.

Als besonders vorteilhaft es sich erwiesen, wenn a) der mindestens eine Extrakt, insbesondere die mindestens zwei Extrakte, von Pflanzen aus der Familie der Heidekrautgewächse (Ericaceae), insbesondere der Gattung Heidelbeere (Vaccinium), den Träger jeweils in Mengen in einem Bereich von 2 bis 70 Gew.-%, insbesondere 3 bis 60 Gew.-%, vorzugsweise 4 bis 55 Gew.-%, bevorzugt 5 bis 50 Gew.-%, bezogen auf die Gesamtzusammensetzung des Extrakts, enthält/enthalten.

Gleichermaßen ist es von Vorteil, wenn b) der mindestens eine Extrakt von Pflanzen aus der Familie der Brennnesselgewächse (Urticaceae) den Träger in Mengen in einem Bereich von 20 bis 70 Gew.-%, insbesondere 25 bis 60 Gew.-%, vorzugsweise 30 bis 55 Gew.-%, bevorzugt 30 bis 50 Gew.-%, bezogen auf die Gesamtzusammensetzung des Extrakts, enthält.

Pflanzenextrakte, die den Träger in den vorgenannten Mengenverhältnissen bzw. -anteilen aufweisen, zeichnen sich durch eine besonders gute Dispergierbarkeit bzw. Löslichkeit in Wasser aus, sodass die Applikation der erfindungsgemäßen Darreichungsform vereinfacht werden kann. Gleichermaßen wird eine gute Stabilisierung der Pflanzenextrakte erreicht, was wiederum vorteilhaft für die Lagerstabilität der Darreichungsform ist und auch die Haltbarkeit bzw. Konservierung der Darreichungsform positiv beeinflusst.

Eine besonders bevorzugte Ausführung der vorliegenden Erfindung sieht dementsprechend vorzugsweise vor, dass a1) der mindestens eine Extrakt von Preiselbeere (*Vaccinium vitis-idaea* L.) jeweils zusammen mit und/oder in Kombination mit einem Träger formuliert ist und/oder vorliegt und/oder dass a2) der mindestens eine Extrakt von Großfrüchtiger Moosbeere (*Vaccinium macrocarpon Aiton*) jeweils zusammen mit und/oder in Kombination mit einem Träger formuliert ist und/oder vorliegt und/oder dass b1) der mindestens eine Extrakt von Brennnesselblättern (*Urticae folium*) jeweils zusammen mit und/oder in Kombination mit einem Träger formuliert ist und/oder vorliegt;
insbesondere wobei der Träger ein wasserlöslicher oder wasserdispergierbarer, insbesondere Kohlenhydrat-basierter und/oder -haltiger, vorzugsweise Oligo- und/oder Polysaccharid-basierter und/oder -haltiger, Träger ist, bevorzugt Maltodextrin ist, und/oder
insbesondere wobei der oder die Extrakte den Träger jeweils in Mengen in einem Bereich von 2 bis 70 Gew.-%, insbesondere 3 bis 60 Gew.-%, vorzugsweise 4 bis 55 Gew.-%, bevorzugt 5 bis 50 Gew.-%, bezogen auf die Gesamtzusammensetzung des Extrakts, enthält/enthalten.

Dabei hat es sich besonders bewährt, wenn a1) der Extrakt von Preiselbeere (*Vaccinium vitis-idaea* L.) den Träger in Mengen in einem Bereich von 2 bis 10 Gew.-%, insbesondere 3 bis 8 Gew.-%, vorzugsweise 4 bis 6 Gew.-%, bevorzugt 5 Gew.-%, bezogen auf die Gesamtzusammensetzung des Extrakts, enthält und/oder
wenn a2) der Extrakt von Großfrüchtiger Moosbeere (*Vaccinium macrocarpon Aiton*) den Träger in Mengen in einem Bereich von 20 bis 70 Gew.-%, insbesondere 25 bis 60 Gew.-%, vorzugsweise 30 bis 55 Gew.-%, bevorzugt 30 bis 50 Gew.-%, bezogen auf die Gesamtzusammensetzung des Extrakts, enthält und/oder
wenn b1) der mindestens eine Extrakt von Brennnesselblättern (*Urticae folium*), den Träger in Mengen in einem Bereich von 20 bis 70 Gew.-%, insbesondere 25 bis 60 Gew.-%, vorzugsweise 30 bis 55 Gew.-%, bevorzugt 30 bis 50 Gew.-%, bezogen auf die Gesamtzusammensetzung des Extrakts, enthält.

Weiter im Hinblick auf die Ausgestaltung und Zusammensetzung der erfindungsgemäß verwendeten Pflanzenextrakte hat es sich im Rahmen der vorliegenden Erfindung als vorteilhaft erwiesen, wenn die Extrakte jeweils als Trockenextrakte ausgebildet sind.

Vorzugsweise ist dabei a) der mindestens eine Extrakt, insbesondere sind die mindestens zwei Extrakte, von Pflanzen aus der Familie der Heidekrautgewächse (Ericaceae), insbesondere der Gattung Heidelbeere (Vaccinium), als Trockenextrakt ausgebildet und/oder ist b) der mindestens eine Extrakt von Pflanzen aus der Familie der Brennnesselgewächse (Urticaceae) als Trockenextrakt ausgebildet.

Die Verwendung von Trockenextrakten trägt vorteilhaft zu einer guten Verarbeitbarkeit der Pflanzenextrakte, insbesondere zusammen mit bzw. in Kombination mit dem vorzugsweise eingesetzten Träger, bei. Weiterhin vorteilhaft erlaubt die Verwendung von Trockenextrakten die effiziente sowie genaue Formulierung und Abmessung der in der Darreichungsform einzusetzenden Extraktmengen. Die eingesetzten Trockenextrakte sind darüber hinaus vorteilhaft besonders kompatibel mit der erfindungsgemäß bevorzugten Formulierung der Darreichungsform als Brausezubereitung, in dem die Trockenextrakte eine sehr gute Re-Dispergierbarkeit aufweisen und somit vorteilhaft zu einer guten Löslichkeit der Darreichungsform in Wasser bzw. auch in Gegenwart von Speichel beitragen. Gleichermaßen ist das gute Löslichkeitsverhalten der Trockenextrakte daher auch für die direkte orale Applikation der erfindungsgemäßen Darreichungsform vorteilhaft.

Dementsprechend ist es im Rahmen der vorliegenden Erfindung weiter bevorzugt vorgesehen, dass a1) der Extrakt von Preiselbeere (*Vaccinium vitis-idaea* L.) als Trockenextrakt ausgebildet ist und/oder dass a2) der Extrakt von Großfrüchtiger Moosbeere (*Vaccinium macrocarpon Aiton*) als Trockenextrakt ausgebildet ist und/oder dass b1) der Extrakt von Brennnesselblättern (*Urticae folium*) als Trockenextrakt ausgebildet ist.

Was nun die Zusammensetzung bzw. Formulierung der erfindungsgemäßen Darreichungsform im Hinblick auf die eingesetzten Extraktmengen anbelangt, so können die Mengenanteile der Pflanzenextrakte üblicherweise in weiten Bereichen variieren. Im Rahmen der vorliegenden Erfindung hat es sich dabei bewährt, wenn die Extrakte in der Darreichungsform jeweils in Mengen in einem Bereich von 5 bis 45 Gew.-%, insbesondere 10 bis 35 Gew.-%, vorzugsweise 15 bis 29 Gew.-%, bevorzugt 17 bis 25 Gew.-%, bezogen auf die Darreichungsform und berechnet auf Basis der Gesamtzusammensetzung der Extrakte, enthalten sind.

Weiter bevorzugt wird es dabei, wenn a) der mindestens eine Extrakt, insbesondere die mindestens zwei Extrakte, von Pflanzen aus der Familie der Heidekrautgewächse (Ericaceae), insbesondere der Gattung Heidelbeere (Vaccinium), in der Darreichungsform in Mengen in einem Bereich von 2 bis 25 Gew.-%, insbesondere 5 bis 20 Gew.-%, vorzugsweise 7 bis 17 Gew.-%, bevorzugt 8 bis 15 Gew.-%, bezogen auf die Darreichungsform und berechnet auf Basis der Gesamtzusammensetzung des Extrakts, enthalten ist/sind und/oder wenn b) der mindestens eine Extrakt von Pflanzen aus der Familie der Brennnesselgewächse (Urticaceae) in der Darreichungsform in Mengen in einem Bereich von 1 bis 20 Gew.-%, insbesondere 3 bis 15 Gew.-%, vorzugsweise 4 bis 12 Gew.-%, bevorzugt 5 bis 10 Gew.-%, bezogen auf die Darreichungsform und berechnet auf Basis der Gesamtzusammensetzung des Extrakts, enthalten ist.

Im Rahmen einer besonders bevorzugten Ausführung der vorliegenden Erfindung hat es sich als vorteilhaft erwiesen, wenn a1) der Extrakt von Preiselbeere (*Vaccinium vitis-idaea* L.) in der Darreichungsform in Mengen in einem Bereich von 1 bis 20 Gew.-%, insbesondere 3 bis 15 Gew.-%, vorzugsweise 4 bis 12 Gew.-%, bevorzugt 5 bis 10 Gew.-%, bezogen auf die Darreichungsform und berechnet auf Basis der Gesamtzusammensetzung des Extrakts, enthalten ist und/oder
wenn a2) der Extrakt von Großfrüchtiger Moosbeere (*Vaccinium macrocarpon Aiton*) in der Darreichungsform in Mengen in einem Bereich von 1 bis 20 Gew.-%, insbesondere 3 bis 15 Gew.-%, vorzugsweise 4 bis 12 Gew.-%, bevorzugt 5 bis 10 Gew.-%, bezogen auf die Darreichungsform und berechnet auf Basis der Gesamtzusammensetzung des Extrakts, enthalten ist und/oder
wenn b1) der Extrakt von Brennnesselblättern (*Urticae folium*) in der Darreichungsform in Mengen in einem Bereich von 1 bis 20 Gew.-%, insbesondere 3 bis 15 Gew.-%, vorzugsweise 4 bis 12 Gew.-%, bevorzugt 5 bis 10 Gew.-%, bezogen auf die Darreichungsform und berechnet auf Basis der Gesamtzusammensetzung des Extrakts, enthalten ist.

Gleichermaßen hat es sich im Hinblick auf die Zusammensetzung der erfindungsgemäßen Darreichungsform, insbesondere für den bevorzugten Fall dass die eingesetzten Pflanzenextrakte als Trockenextrakte vorliegen bzw. zusammen mit und/oder in Kombination mit einem Träger eingesetzt werden, bewährt, wenn die Extrakte in der Darreichungsform in Mengen in einem Bereich von 2 bis 30 Gew.-%, insbesondere 4 bis 22 Gew.-%, vorzugsweise 6 bis 19 Gew.-%, bevorzugt 7 bis 17 Gew.-%, bezogen auf die Darreichungsform und berechnet auf Basis der Extraktzusammensetzung ohne Träger und/oder auf Basis der eingesetzten Trockenextraktmenge, enthalten sind.

Im Rahmen einer diesbezüglichen vorteilhaften Weiterbildung hat es sich als vorteilhaft erwiesen, wenn a) der mindestens eine Extrakt, insbesondere die mindestens zwei Extrakte, von Pflanzen aus der Familie der Heidekrautgewächse (Ericaceae), insbesondere der Gattung Heidelbeere (Vaccinium), in der Darreichungsform in Mengen in einem Bereich von 3 bis 20 Gew.-%, insbesondere 5 bis 15 Gew.-%, vorzugsweise 7 bis 13 Gew.-%, bevorzugt 8 bis 12 Gew.-%, bezogen auf die Darreichungsform und berechnet auf Basis der Extraktzusammensetzung ohne Träger und/oder auf Basis der eingesetzten Trockenextraktmenge, enthalten ist/sind und/oder
wenn b) der mindestens eine Extrakt von Pflanzen aus der Familie der Brennnesselgewächse (Urticaceae) in der Darreichungsform in Mengen in einem Bereich von 0,5 bis 10 Gew.-%, insbesondere 1,5 bis 7 Gew.-%, vorzugsweise 2 bis 6 Gew.-%, bevorzugt 3 bis 5 Gew.-%, bezogen auf die Darreichungsform und berechnet auf Basis der Extraktzusammensetzung ohne Träger und/oder auf Basis der eingesetzten Trockenextraktmenge, enthalten ist.

Noch weiter bevorzugt wird es, wenn a1) der Extrakt von Preiselbeere (*Vaccinium vitis-idaea* L.) in der Darreichungsform in Mengen in einem Bereich von 2 bis 15 Gew.-%, insbesondere 3 bis 12 Gew.-%, vorzugsweise 4 bis 10 Gew.-%, bevorzugt 5 bis 8 Gew.-%, bezogen auf die Darreichungsform und berechnet auf Basis der Extraktzusammensetzung ohne Träger und/oder auf Basis der eingesetzten Trockenextraktmenge, enthalten ist und/oder
wenn a2) der Extrakt von Großfrüchtiger Moosbeere (*Vaccinium macrocarpon Aiton*) in der Darreichungsform in Mengen in einem Bereich von 0,5 bis 10 Gew.-%, insbesondere 1,5 bis 7 Gew.-%, vorzugsweise 2 bis 6 Gew.-%, bevorzugt 3 bis 5 Gew.-%, bezogen auf die Darreichungsform und berechnet auf Basis der Extraktzusammensetzung ohne Träger und/oder gegenüber der eingesetzten Trockenextraktmenge, enthalten ist und/oder
wenn b1) der Extrakt von Brennnesselblättern (*Urticae folium*) in der Darreichungsform in Mengen in einem Bereich von 0,5 bis 10 Gew.-%, insbesondere 1,5 bis 7 Gew.-%, vorzugsweise 2 bis 6 Gew.-%, bevorzugt 3 bis 5 Gew.-%, bezogen auf die Darreichungsform und berechnet auf Basis der Extraktzusammensetzung ohne Träger und/oder auf Basis der eingesetzten Trockenextraktmenge, enthalten ist.

Weiter im Hinblick auf die Zusammensetzung sowie insbesondere das Verhältnis der verwendeten Komponenten in der Darreichungsform zueinander hat es sich im Rahmen der vorliegenden Erfindung als vorteilhaft erwiesen, wenn a) der mindestens eine Extrakt, insbesondere die mindestens zwei Extrakte, von Pflanzen aus der Familie der Heidekrautgewächse (Ericaceae), insbesondere der Gattung Heidelbeere (Vaccinium), und b) der mindestens eine Extrakt von Pflanzen aus der Familie der Brennnesselgewächse (Urticaceae) in der Darreichungsform in einem Mengenverhältnis in einem Bereich a) : b) von 10 : 1 bis 1 : 10, insbesondere 7 : 1 bis 1 : 5, vorzugsweise 5 : 1 bis 1 : 2, bevorzugt 2,5 : 1 bis 1 : 1, jeweils bezogen auf die Gesamtzusammensetzung der Extrakte, enthalten sind.

Noch weiter bevorzugt ist es, wenn a) der mindestens eine Extrakt, insbesondere die mindestens zwei Extrakte, von Pflanzen aus der Familie der Heidekrautgewächse (Ericaceae), insbesondere der Gattung Heidelbeere (Vaccinium), und b) der mindestens eine Extrakt von Pflanzen aus der Familie der Brennnesselgewächse (Urticaceae) in der Darreichungsform in einem Mengenverhältnis in einem Bereich von 15: 1 bis 1 : 7, insbesondere 10: 1 bis 1 : 2, vorzugsweise 6 : 1 bis 1 : 1, bevorzugt 4 : 1 bis 2 : 1, jeweils bezogen auf die Extraktzusammensetzung ohne Träger und/oder die eingesetzte Trockenextraktmenge, enthalten sind.

Im Rahmen einer bevorzugten Ausführung der vorliegenden Erfindung ist es vorgesehen, dass a1) der Extrakt von Preiselbeere (*Vaccinium vitis-idaea* L.), a2) der Extrakt von Großfrüchtiger Moosbeere (*Vaccinium macrocarpon Aiton*) und b1) der Extrakt von Brennnesselblättern (*Urticae folium*) in der Darreichungsform in einem Mengenverhältnis in einem Bereich von 10 : 10 : 1 bis 1 : 1 : 10, insbesondere 7 : 7 : 1 bis 1 : 1 : 5, vorzugsweise 5 : 5 : 1 bis 1 : 1 : 2, bevorzugt 2,5 : 2,5 : 1 bis 1 : 1 : 1,5, jeweils bezogen auf die Gesamtzusammensetzung der Extrakte, enthalten sind.

Demgegenüber noch weiter bevorzugt ist es, wenn im Rahmen der vorliegenden Erfindung a1) der Extrakt von Preiselbeere (*Vaccinium vitis-idaea* L.), a2) der Extrakt von Großfrüchtiger Moosbeere (*Vaccinium macrocarpon Aiton*) und b1) der Extrakt von Brennnesselblättern (*Urticae folium*) in der Darreichungsform in einem Mengenverhältnis in einem Bereich von 9 : 1 : 1 bis 1 : 3 : 3, insbesondere 6 : 1 : 1 bis 1 : 1,5 :1,5, vorzugsweise 4 : 1 : 1 bis 1 : 1 : 1, bevorzugt 2 : 1 : 1 bis 1,5 : 1 : 1, jeweils bezogen auf die Extraktzusammensetzung ohne Träger und/oder die eingesetzte Trockenextraktmenge, enthalten sind.

In den vorgenannten Mengenbereichen bzw. Mengenverhältnissen für die eingesetzten Pflanzenextrakte sind die mit der vorliegenden Erfindung assoziierten Vorteile und besonderen Wirkungen besonders ausgeprägt und können Darreichungsformen mit einem besonders effektiven Wirkprofil bzw. einer besonders vorteilhaften ergänzenden Wirkung als Nahrungsergänzungsmittel erhalten werden. Gleichermaßen können Darreichungsformen erhalten werden, die sich im Hinblick auf die sensorischen Eigenschaften, beispielsweise insbesondere den Geschmack, Geruch, das Mund- und Schluckgefühl, sowie auch die optische Gefälligkeit, durch ein insgesamt als positives zu bewertendes Eigenschaftsprofil auszeichnen. Besonders vorteilhaft können in den vorgenannten Mengenbereichen bzw. - verhältnissen also Darreichungsformen erhalten werden, die neben einer verlässlichen therapeutischen Wirksamkeit bzw. einer vorteilhaften Eignung als Nahrungsergänzungsmittel auch einen sehr angenehmen Geschmack ebenso wie einen angenehmen Geruch und auch eine optisch ansprechende Erscheinung aufweisen, so dass neben der eigentlichen therapeutischen bzw. nahrungsergänzenden Wirkung auch ein positives sensorisches Empfinden erzielt wird, was insbesondere der Applikation und diesbezüglichen Motivation von Patient bzw. Konsument zuträglich ist.

Was nun weiter die inhaltliche Zusammensetzung der Pflanzenextrakte, die in erfindungsgemäßen Darreichungsformen eingesetzt werden, anbelangt, so ist es im Allgemeinen vorgesehen, dass diese ein Droge/Extrakt-Verhältnis (DEV) in einem fachüblichen Bereich aufweisen. Als vorteilhaft erwiesen hat es sich, wenn die Extrakte ein Droge/Extrakt-Verhältnis (DEV) in einem Bereich von 75 : 1 bis 1 : 10, insbesondere 65 : 1 bis 1 : 5, vorzugsweise 60 : 1 bis 1 : 1, bevorzugt 55 : 1 bis 3 : 1, jeweils bezogen auf die Gesamtzusammensetzung der Extrakte, aufweisen.

Das Droge/Extrakt-Verhältnis (DEV) beschreibt dabei das Verhältnis aus Masse der eingesetzten Droge, d.h. der jeweils eingesetzten Rohstoffmenge der für die eingesetzten Extrakte verwendeten Pflanzen, gegenüber dem gewonnenen, nativen Extrakt ohne weitere Hilfsstoffe bzw. ggf. Träger oder dgl.

Die im Rahmen der vorliegenden Erfindung bevorzugt angestrebten Droge/Extrakt-Verhältnisse garantieren dabei vorteilhaft, dass die mit der erfindungsgemäßen Darreichungsform assoziierten Vorteile und besonderen technischen Wirkungen, insbesondere die vorteilhaften physiologischen bzw. therapeutischen Wirkungen, effizient erreicht werden können, insbesondere da teils hochwirksame bzw. hochkonzentrierte Extrakte eingesetzt werden. Die Verwendung von Pflanzenextrakten, welche die vorgenannten Droge/Extrakte-Verhältnisse aufweisen, gestattet außerdem eine effiziente Formulierung mit relativ reduzierten Dosismengen, insbesondere da wirkkonzentriertere Ausgangstoffe verwendet werden, sodass vorteilhaft letztlich auch mengen -bzw. gewichtsoptimierte Dosis- bzw. Produkteinheiten der erfindungsgemäßen Darreichungsform bereitgestellt werden können.

In einer bevorzugten Ausgestaltung der vorliegenden Erfindung hat es sich bewährt, wenn a) der mindestens eine Extrakt, insbesondere die mindestens zwei Extrakte, von Pflanzen aus der Familie der Heidekrautgewächse (Ericaceae), insbesondere der Gattung Heidelbeere (Vaccinium), ein Droge/Extrakt-Verhältnis (DEV) in einem Bereich von 75 : 1 bis 1 : 10, insbesondere 65 : 1 bis 1 : 5, vorzugsweise 60 : 1 bis 1 : 1, bevorzugt 55 : 1 bis 3 : 1, bezogen auf die Gesamtzusammensetzung der Extrakte, aufweist/aufweisen und/oder
wenn b) der mindestens eine Extrakt von Pflanzen aus der Familie der Brennnesselgewächse (Urticaceae) ein Droge/Extrakt-Verhältnis (DEV) in einem Bereich von 20 : 1 bis 1 :10, insbesondere 15:1 bis 1 : 5, vorzugsweise 10:1 bis 1 : 1, bevorzugt 7 : 1 bis 3 : 1, bezogen auf die Gesamtzusammensetzung der Extrakte, aufweist.

Noch weiter bevorzugt ist es im Raum der vorliegenden Erfindung, wenn a1) der mindestens eine Extrakt von Preiselbeere (*Vaccinium vitis-idaea* L.) ein Droge/Extrakt-Verhältnis (DEV) in einem Bereich von 75 : 1 bis 35 : 1, insbesondere 65 : 1 bis 40 : 1, vorzugsweise 60 : 1 bis 43 : 1, bevorzugt 55 : 1 bis 45 : 1, bezogen auf die Gesamtzusammensetzung des Extrakts, aufweist und/oder
wenn a2) der mindestens eine Extrakt von Großfrüchtiger Moosbeere (*Vaccinium macrocarpon Aiton*) ein Droge/Extrakt-Verhältnis (DEV) in einem Bereich von 20 : 1 bis 1 : 10, insbesondere 15 : 1 bis 1 : 5, vorzugsweise 10 : 1 bis 1 : 1, bevorzugt 7 : 1 bis 3 : 1, bezogen auf die Gesamtzusammensetzung des Extrakts, aufweist und/oder
wenn b1) der mindestens eine Extrakt von Brennnesselblättern (*Urticae folium*) ein Droge/Extrakt-Verhältnis (DEV) in einem Bereich von 20 : 1 bis 1 : 10, insbesondere 15 : 1 bis 1 : 5, vorzugsweise 10 : 1 bis 1 : 1, bevorzugt 7 : 1 bis 3 : 1, bezogen auf die Gesamtzusammensetzung des Extrakts, aufweist.

Darüber hinaus ist es hinsichtlich des Wirkstoffgehalts der erfindungsgemäßen Darreichungsform, insbesondere im Hinblick auf den Gehalt der Darreichungsform an Proanthocyanidinen, im Rahmen der vorliegenden Erfindung bevorzugt, wenn die Darreichungsform einen Proanthocyanidin-Gehalt in einem Bereich von 0,01 bis 5 Gew.-%, insbesondere 0,05 bis 3,5 Gew.-%, vorzugsweise 0,1 bis 3 Gew.-%, bevorzugt 0,5 bis 2,5 Gew.-%, bezogen auf die Darreichungsform, aufweist.

Im Rahmen einer bevorzugten Ausführung der erfindungsgemäßen Darreichungsform hat es sich bewährt, wenn a) der mindestens einen Extrakt, insbesondere die mindestens zwei Extrakte, von Pflanzen aus der Familie der Heidekrautgewächse (Ericaceae), insbesondere der Gattung Heidelbeere (Vaccinium), insbesondere a1) der mindestens eine Extrakt von Preiselbeere (*Vaccinium vitis-idaea*), vorzugsweise die Darreichungsform, einen Proanthocyanidin-Gehalt in einem Bereich von 0,01 bis 5 Gew.-%, insbesondere 0,05 bis 3,5 Gew.-%, vorzugsweise 0,1 bis 3 Gew.-%, bevorzugt 0,5 bis 2,5 Gew.-%, bezogen auf die Gesamtzusammensetzung des Extrakts, vorzugsweise bezogen auf die Darreichungsform, aufweist/aufweisen;
insbesondere wobei der Proanthocyanidin-Gehalt der Darreichungsform zumindest im Wesentlichen dem Proanthocyanidin-Gehalt a) des mindestens einen Extrakts, insbesondere der mindestens zwei Extrakte, von Pflanzen aus der Familie der Heidekrautgewächse (Ericaceae), insbesondere der Gattung Heidelbeere (Vaccinium), insbesondere a1) des mindestens einen Extrakts von Preiselbeere (*Vaccinium vitis-idaea*), entspricht.

Wenn der Proanthocyanidin-Gehalt der erfindungsgemäßen Darreichungsform in den vorgenannten Mengenbereichen liegt, werden vorteilhaft besonders wirkungsvolle Darreichungsformen erhalten, insbesondere wobei die mit der erfindungsgemäßen Darreichungsform assoziierten Vorteile und besonderen technischen Wirkungen, insbesondere die vorteilhaften physiologischen bzw. therapeutischen Wirkungen, effizient erreicht werden. Gleichfalls kann eine mengen- bzw. gewichtsoptimierte Formulierung der erfindungsgemäßen Darreichungsform umgesetzt werden, sodass Dosierungs- bzw. Produkteinheiten mit optimierten Mengen bzw. Volumina bereitgestellt werden können, insbesondere wobei der Extraktanteil mengenoptimiert eingesetzt werden kann.

Weiterhin hat es sich im Rahmen der vorliegenden Erfindung bewährt, wenn die diuretisch-wirksame und/oder entzündungshemmende Wirkstoffkomponente, insbesondere b), vorzugsweise b1), Kaffeoyläpfelsäure und/oder Chlorogensäure als Wirkstoff enthält.

Die beiden vorgenannten Wirkstoffe sind vorteilhaft für die diuretische sowie auch entzündungshemmende Wirkung der erfindungsgemäßen Darreichungsform maßgeblich. Insofern tragen die vorgenannten Wirkstoffe zu einer milden Steigerung des Hahnflusses bei, was sowohl für die therapeutische als auch die prophylaktische Behandlung von Harnwegsinfektionen als auch im Zusammenhang mit der nahrungsergänzenden Anwendung der erfindungsgemäßen Darreichungsform zum Erhalt bzw. zur Sicherstellung der Blasengesundheit und der normalen Funktion des Urgenitaltraktes vorteilhaft ist, insbesondere indem eine bessere Spülung der Blase initiiert und ein weniger entzündliches Blasenmilieu erzeugt wird.

Im Rahmen der vorliegenden Erfindung hat es sich außerdem bewährt, wenn die Darreichungsform mindestens einen weiteren Pflanzenextrakt, insbesondere von Pflanzen aus der Familie der Korbblütler (Asteraceae), vorzugsweise der Gattung Goldrute (Solidago), umfasst.

Bevorzugt ist der weitere Extrakt dabei ausgewählt aus der Gruppe von Gewöhnlicher Goldrute (*Solidago virgaurea*), Riesen-Goldrute (*Solidago gigantea*), Kanadischer Goldrute (Solidago canadensis), Hain-Goldrute (*Solidago nemoralis*), insbesondere Gewöhnlicher Goldrute (S*olidago virgaurea*), Riesen-Goldrute (*Solidago gigantea*), Kanadischer Goldrute (Solidago canadensis), vorzugsweise Gewöhnlicher Goldrute (*Solidago virgaurea*), Riesen-Goldrute (*Solidago gigantea*).

Besonders gute Ergebnisse werden dabei erhalten, wenn der weitere Extrakt in der Darreichungsform in Mengen in einem Bereich von 0,1 bis 15 Gew.-%, insbesondere 0,5 bis 12,5 Gew.-%, vorzugsweise 1 bis 11 Gew.-%, bevorzugt 1,5 bis 10 Gew.-%, bezogen auf die Darreichungsform und berechnet auf Basis der Gesamtzusammensetzung, enthalten ist.

Gleichermaßen hat es sich als geeignet erwiesen, wenn der weitere Extrakt zusammen mit und/oder in Kombination mit einem Träger formuliert ist und/oder vorliegt;
insbesondere wobei der Träger ein wasserlöslicher oder wasserdispergierbarer, insbesondere Kohlenhydrat-basierter und/oder -haltiger, vorzugsweise Oligo- und/oder Polysaccharid-basierter und/oder -haltiger, Träger ist, bevorzugt Maltodextrin ist, und/oder
insbesondere wobei der Extrakt den Träger in Mengen in einem Bereich von 20 bis 70 Gew.-%, insbesondere 25 bis 60 Gew.-%, vorzugsweise 30 bis 55 Gew.-%, bevorzugt 30 bis 50 Gew.-%, bezogen auf die Gesamtzusammensetzung des Extrakts, enthält.

Außerdem ist es bevorzugt, wenn der weitere Extrakt ein Droge/Extrakt-Verhältnis (DEV) in einem Bereich von 20: 1 bis 1 : 10, insbesondere 15: 1 bis 1 : 5, vorzugsweise 10 : 1 bis 1 : 1, bevorzugt 7:1 bis 3:1, bezogen auf die Gesamtzusammensetzung des Extrakts, aufweist.

Die Verwendung eines weiteren Extraktes, insbesondere von von Pflanzen aus der Familie der Korbblütler (Asteraceae), vorzugsweise der Gattung Goldrute (Solidago) komplementiert die Wirksamkeit der erfindungsgemäßen Darreichungsform bzw. den physiologischen Effekt dieser für die vorgesehenen Verwendungen und Indikationen in vorteilhafter Weise, besonders in den genannten Mengen bzw. für Extrakte, die wie beschrieben, formuliert sind.

Wie bereits eingangs ausgeführt, liegt die erfindungsgemäße Darreichungsform vorzugsweise in Form einer Brausezubereitung vor. In diesen Zusammenhang hat es sich nun als vorteilhaft ergeben, wenn die Brausezubereitung einen Brausesatz umfasst. Dabei hat es sich bewährt, wenn der Brausesatz mindestens einen Kohlendioxid-freisetzenden Inhaltsstoff, vorzugsweise ausgewählt aus der Gruppe von Alkali- und/oder Erdalkalicarbonaten und -hydrogencarbonaten sowie deren Kombinationen, und mindestens einen mit dem Kohlendioxid-freisetzenden Inhaltsstoff reagierenden Inhaltsstoff, vorzugsweise eine schwache und/oder organische Säure, bevorzugt ausgewählt aus der Gruppe von Äpfelsäure, Zitronensäure, Weinsäure sowie deren Kombinationen, enthält.

Ein entsprechender Brausesatz geht mit hervorragenden Löslichkeitseigenschaften sowohl bei der Applikation der Darreichungsform als wässrigen Lösung, d.h. in Form eines Brausegetränks, als auch bei einer direkten oralen Einnahme der Darreichungsform einher, indem vorteilhaft ein schnelles Auflösen der Darreichungsform sichergestellt wird.

Erfindungsgemäß hat es sich dabei besonders bewährt, wenn der Brausesatz den Kohlendioxid-freisetzenden Inhaltsstoff und den mit dem Kohlendioxid-freisetzenden Inhaltsstoff reagierenden Inhaltsstoff in einem Verhältnis in einem Bereich von 5 : 1 bis 1 : 0,25, insbesondere 4 : 1 bis 1 : 0,5, vorzugsweise 3: 1 bis 1 : 1, bevorzugt 2,5 : 1 bis 1,5 : 1, enthält.

Weiterhin hat es sich im Rahmen der vorliegenden Erfindung als vorteilhaft erwiesen, wenn die erfindungsgemäße Darreichungsform den Brausesatz in Mengen in einem Bereich von 20 bis 60 Gew.-%, insbesondere 22,5 bis 50 Gew.-%, vorzugsweise 25 bis 45 Gew.-%, bevorzugt 25,7 bis 40 Gew.-%, enthält.

Gute Ergebnisse werden im Rahmen der vorliegenden Erfindung außerdem erzielt, wenn die Darreichungsform ein antibakteriell-wirksames Saccharid, insbesondere Monosaccharid, vorzugsweise Mannose, besonders bevorzugt D-Mannose, umfasst.

Hierbei hat es sich bewährt, wenn die Darreichungsform das Saccharid in Mengen in einem Bereich von 0,0001 bis 10 Gew.-%, insbesondere 0,001 bis 7,5 Gew.-%, vorzugsweise 0,01 bis 5 Gew.-%, bevorzugt 0,1 bis 3 Gew.-%, bezogen auf die Darreichungsform, enthält.

Wenn die Darreichungsform nach der vorliegenden Erfindung ein antibakteriell-wirksames Saccharid, insbesondere Monosaccharid, vorzugsweise Mannose, besonders bevorzugt D-Mannose enthält, wird vorteilhaft eine noch ausgeprägtere Wirkung der erfindungsgemäßen Darreichungsform im Hinblick auf die bevorzugte Indikation bzw. die vorgesehenen Verwendungen erreicht, insbesondere in den vorgenannten Mengenbereichen.

Weiter im Hinblick auf die Zusammensetzung der erfindungsgemäßen Darreichungsform kann es sich außerdem anbieten, der Darreichungsform weitere Inhaltsstoffe, die physiologisch wertvoll sind bzw. eine physiologische Aktivität aufweisen, hinzuzufügen. So hat es sich etwa bewährt, wenn die Darreichungsform mindestens einen Mineralstoff und/oder mindestens ein Vitamin, vorzugsweise mindestens einen Mineralstoff und mindestens ein Vitamin, umfasst;
insbesondere wobei der Mineralstoff ausgewählt ist aus der Gruppe von physiologisch kompatiblen Magnesium-, Kalium und/oder Calcium-Salzen, insbesondere physiologisch kompatiblen Magnesium-, Kalium und/oder Calcium-Salzen organischer Säuren, vorzugsweise Magnesium-, Kalium und/oder Calcium-Citraten, bevorzugt Trimagnesiumdicitrat, und/oder
insbesondere wobei die Darreichungsform den Mineralstoff in Mengen in einem Bereich von 25 bis 40 Gew.-%, insbesondere 27,5 bis 37,5 Gew.-%, vorzugsweise 29 bis 35 Gew.-%, bevorzugt 30 bis 34 Gew.-%, bezogen auf die Darreichungsform, enthält und/oder
insbesondere wobei das Vitamin ausgewählt ist aus der Gruppe von Vitamin C, Vitamin A, Vitamin D, Vitamin E, B-Vitaminen, insbesondere Vitamin C, und/oder
insbesondere wobei die Darreichungsform das Vitamin in Mengen in einem Bereich von 0,1 bis 5 Gew.-%, insbesondere 0,25 bis 3 Gew.-%, vorzugsweise 0,5 bis 2 Gew.-%, bevorzugt 0,75 bis 1,5 Gew.-%, bezogen auf die Darreichungsform, enthält.

Die Verwendung von Mineralstoffen wie insbesondere Magnesium, insbesondere in den erfindungsgemäß vorgesehenen Mengen, kann einen vorteilhaften Beitrag zum Elektrolytgleichgewicht des Körpers leisten. Darüber hinaus kann insbesondere Magnesium in höheren Mengen, insbesondere ab einer täglich aufgenommen Mindestmenge von 200 mg, entzündungshemmend sowie krampfreduzierend wirken. Diese krampfreduzierende Wirkung kann insbesondere im Zusammenhang mit der therapeutischen Anwendung der erfindungsgemäßen Darreichungsform eine gewisse Linderung für die Abgabe von Urin verschaffen, indem diese zumindest geringfügig erleichtert werden bzw. weniger unangenehm sein kann. Die vorgenannten vorteilhaften Effekten können insbesondere auch dann beobachtet werden, wenn die erfindungsgemäße Darreichungsform, insbesondere eine Produkteinheit bzw. Dosiereinheit dieser, mehrmals täglich eingenommen wird. In diesen Fall trägt der erfindungsgemäß bevorzugt vorgesehene Magnesiumgehalt sowohl zu einem normalen Elektrolytgleichgewicht als auch zu einem vorteilhaften Effekt auf die Muskelphysiologie bei.

Vitamin C dient vorteilhaft der Erhöhung der Eisenaufnahme und trägt insbesondere zu einer normalen Funktion des Immunsystems bei, was im Zusammenhang mit der weiteren Wirkweise bzw. den vorteilhaften physiologischen bzw. therapeutischen Effekten der erfindungsgemäßen Darreichungsform einen kombinatorischen bzw. insbesondere synergistischen Effekt darstellt, denn insbesondere für die prophylaktische bzw. therapeutische Behandlung von Infektionskrankheiten ist eine zusätzliche Stärkung des Immunsystems wünschenswert, und auch für die Förderung bzw. den Erhalt der Blasengesundheit sind ein normales Immunsystem sowie Maßnahmen zu dessen Erhalt insgesamt zu begrüßen.

Darüber hinaus hat sich im Rahmen der vorliegenden Erfindung als vorteilhaft erwiesen, wenn die Darreichungsform mindestens ein Sprengmittel und/oder einen Zerfallsbeschleuniger umfasst;
insbesondere wobei das Sprengmittel und/oder der Zerfallsbeschleuniger ausgewählt ist aus der Gruppe von Stärke und Stärkeethern, Cellulose, vernetzter Cellulose, insbesondere Alkalisalzen von vernetzter Cellulose, vorzugsweise Alkalisalzen von vernetzter Carboxymethylcellulose, bevorzugt vernetzter Natriumcarboxymethylcellulose bzw. Croscarmellose; Alginsäure und deren Salze, Polyvinylpyrrolidonen, insbesondere quervernetzte Polyvinylpyrrolidonen, und/oder
insbesondere wobei die Darreichungsform das Sprengmittel und/oder den Zerfallsbeschleuniger in Mengen in einem Bereich von 0,1 bis 10 Gew.-%, insbesondere 0,5 bis 8 Gew.-%, vorzugsweise 0,75 bis 6,5 Gew.-%, bevorzugt 1 bis 5 Gew.-%, bezogen auf die Darreichungsform, enthält

Erfindungsgemäß ist dabei insbesondere die Verwendung von Natriumcarboxymethylcellulose bzw. Croscarmellose als Zerfallsbeschleuniger bevorzugt, insbesondere wobei die erfindungsgemäße Zusammensetzung ganz besonders bevorzugt 1,5 bis 4 Gew.-% der Natriumcarboxymethylcellulose bzw. Croscarmellose enthält.

Der Zusatz von Sprengmitteln und/oder der Zerfallsbeschleunigern, insbesondere von vernetzter Natriumcarboxymethylcellulose bzw. Croscarmellose-Natrium hat vorteilhaft den Effekt, dass Löseverhalten der erfindungsgemäßen Darreichungsform noch weiter zu verbessern und insbesondere die Dauer, in der sich eine Packungs- bzw. Dosiereinheit der Darreichungsform in wässriger Lösung auflöst zu verkürzen. So kann die Applikation der Darreichungsform, insbesondere sowohl als Brausetablette als auch als Brausepulver, noch weiter vereinfacht und eine gute und schnelle Löslichkeit der Darreichungsform sichergestellt werden. Erfindungsgemäß bevorzugte Sprengmittel bzw. Zerfallsbeschleuniger sind dabei selbst regelmäßig wasserunlöslich, neigen jedoch dazu, in Gegenwart von Wasser stark aufzuquellen, so dass infolge der Volumenexpansion des Sprengmittels und einem entsprechenden Anstieg internen Drucks insbesondere eine Darreichungsform in Form einer Brausetablette deutlich schneller desintegriert als vergleichbare Zusammensetzungen ohne Sprengmittel.

Weiter hat es sich im Rahmen der vorliegenden Erfindung bewährt, wenn die Darreichungsform mindestens einen weiteren Inhaltsstoff und/oder mindestens einen weiteren Hilfsstoff umfasst;
insbesondere wobei der mindestens eine Inhaltsstoff und/oder der mindestens eine Hilfsstoff ausgewählt ist aus der Gruppe von Füll-, Streck-, Binde-, Netz-, Stabilisierungs-, Färbe-, Puffer-, Riech-, Geschmacks-, Süßungs-, Aroma-, Nähr-, Gleit-, Verarbeitungshilfs- und/oder Konservierungsstoffen und -mitteln, vorzugsweise Vitaminen, insbesondere Vitamin A, Vitamin D, Vitamin E, B-Vitaminen und/oder deren Mischungen; Mineralstoffen, insbesondere Magnesium-, Kalium und/oder Calcium-Citraten; Süßstoffen, insbesondere Sucralose; Aromen, insbesondere Fruchtaromen, vorzugsweise Himbeer-Aroma; Zuckeraustauschstoffen, insbesondere Isomalt; Zuckeralkoholen, Füllstoffen, Bindemitteln, insbesondere Hydroxypropylcellulose und/oder niedrig-substituierter Hydroxypropylcellulose (L-HPC);
bevorzugt wobei die weiteren Inhaltsstoffe ausgewählt sind aus Vitamin A, Vitamin D, Vitamin E, B-Vitaminen und/oder deren Mischungen, Magnesium-, Kalium und/oder Calcium-Citraten, Sucralose, Himbeer-Aroma, Isomalt, Hydroxypropylcellulose und/oder niedrig-substituierter Hydroxypropylcellulose (L-HPC).

Gute Ergebnisse werden dabei im Rahmen der vorliegenden Erfindung erhalten, wenn die Darreichungsform den mindestens einen Inhaltsstoff und/oder den mindestens einen Hilfsstoff in Mengen in einem Bereich von bis zu 55 Gew.-%, insbesondere bis zu 50 Gew.-%, vorzugsweise bis zu 47 Gew.-%, bevorzugt bis zu 45 Gew.-%, bezogen auf die Darreichungsform, enthält.

Im Rahmen der vorliegenden Erfindung ist es dabei ganz besonders bevorzugt, wenn die erfindungsgemäße Darreichungsform als weiteren Inhaltsstoff und/oder der Hilfsstoff zumindest Hydroxypropylcellulose und insbesondere niedrig-substituierte Hydroxypropylcellulose (L-HPC) enthält. Besonders vorteilhaft ist dabei die Verwendung von niedrig-substituierte Hydroxypropylcellulose (L-HPC) in Kombination mit dem Zerfallsbeschleuniger Natriumcarboxymethylcellulose bzw. Croscarmellose. Für die Kombination können sehr kurze Zerfallszeiten bzw. Auflösezeiten beobachtet werden, wenn die erfindungsgemäße Zusammensetzung als Brausezubereitung ausgebildet ist. In diesem Fall hat es sich als besonders vorteilhaft erwiesen, wenn die erfindungsgemäße Darreichungsform Hydroxypropylcellulose und insbesondere niedrig-substituierte Hydroxypropylcellulose (L-HPC) in Mengen in einem Bereich von 0,1 bis 25 Gew.-%, insbesondere 0,5 bis 15 Gew.-%, vorzugsweise 0,75 bis 10 Gew.-%, bevorzugt 1 bis 5 Gew.-%, enthält.

Weiterhin ist es für die Formulierung bzw. Galenik der erfindungsgemäßen Darreichungsform vorzugsweise vorgesehen, dass die erfindungsgemäße Darreichungsform, insbesondere die Wirk- und/oder Inhaltsstoffe der Darreichungsform zusammen mit und/oder in Kombination mit mindestens einem Exzipienten, insbesondere einem physiologisch kompatiblen Exzipienten, vorzugsweise einem Nahrungsergänzungsmittel- und/oder pharmazeutischen Exzipienten, formuliert sind und/oder vorliegen.

Was nun die Zubereitung der erfindungsgemäßen Darreichungsform anbelangt, so hat es sich im Rahmen der vorliegenden Erfindung besonders bewährt, wenn die Darreichungsform als Brausezubereitung, insbesondere in Form eines losen oder gepressten Pulvers, vorzugsweise in Form einer Brausetablette, formuliert ist und/oder vorliegt;
insbesondere wobei die Darreichungsform in Form einer Brausetablette zumindest im Wesentlichen kugelförmig, insbesondere in Form einer insbesondere oval verzerrten Kugel oder eines Ellipsoids jeweils mit mittig umlaufendem Steg, ausgebildet ist,
vorzugsweise wobei die kugelförmig, insbesondere in Form einer insbesondere oval verzerrten Kugel oder eines Ellipsoids, ausgebildete Brausetablette einen mittig umlaufenden Steg aufweist, insbesondere wobei der mittig umlaufende Steg einen zumindest im Wesentlichen zylindrischen Abschnitt ausbildet, vorzugsweise sodass eine Braustablette in Form zweier zusammengesetzter, durch einen insbesondere zylindrisch geformten, mittig umlaufenden Steg verbundener, Halbkugeln ausgebildet ist.

Die Zubereitung der Darreichungsform als Brausetablette mit einer insbesondere zumindest im wesentlichen kugelförmigen Ausgestaltung zeichnet sich vorteilhaft dadurch aus, dass für eine feste Darreichungsform ein nahezu optimales Oberfläche-/Volumen-Verhältnis erreicht wird, sodass eine im Hinblick auf den Umfang bzw. die Größe der Dosier- bzw. Packungseinheit möglichst effiziente, kleinvolumige Formulierung einer Dosis der Darreichungsform möglich ist. Vorteilhaft sowie bevorzugt weist die erfindungsgemäße Darreichungsform in Form dieser bevorzugten Zubereitung bzw. Ausführung als Brausetablette eine Härte von mehr als 20 N (Newton), insbesondere mehr als 25 N, vorzugsweise mehr als 30 N, bevorzugt mehr als 35 N, auf. Besonders bevorzugt weist die erfindungsgemäße Darreichungsform eine Härte in einem Bereich von 20 N bis 70 N, insbesondere 25 N bis 60 N, vorzugsweise 30 N bis 50 N, bevorzugt 35 N bis 45 N, auf. Die Bestimmung der Härte von Brausetabletten der erfindungsgemäßen Darreichungsform erfolgt dabei in Übereinstimmung mit den Vorgaben gemäß *Ph. Eur. 2.9.8*. (*European Pharmacopoeia 9*.*0*).

Darüber hinaus wird für die Ausgestaltung als Brausetablette ebenfalls ein sehr gutes sowie auch charakteristisches Löslichkeitsverhalten der Darreichungsform in wässriger Lösung beobachtet. Es wird vorteilhaft sowohl ein gleichmäßiges und zügiges sowie vollständiges und rückstandsfreies Auflösen der Brausetablette erreicht. Auf Grund der bevorzugten Ausgestaltung insbesondere in Form einer insbesondere oval verzerrten Kugel oder eines Ellipsoids, ausgebildeten Brausetablette, die vorzugsweise zudem einen mittig umlaufenden Steg aufweist, insbesondere wobei der mittig umlaufende Steg einen zumindest im Wesentlichen zylindrischen Abschnitt ausbildet, vorzugsweise sodass eine Braustablette in Form zweier zusammengesetzter, durch einen insbesondere zylindrisch geformten, mittig umlaufenden Steg verbundener, Halbkugeln ausgebildet ist, schwimmt die Darreichungsform beim Auflösen in Wasser auf der Wasseroberfläche auf und führt eine als "tanzend" anmutende bzw. interpretierbare Taumelbewegung auf der Wasseroberfläche aus. Vorteilhaft kann so verfolgt werden, ob bzw. dass sich die Tablette rückstandsfrei sowie vollständig auflöst. Außerdem kann beim Patienten bzw. Konsumenten ein visuell anregendes sowie auch zur Einnahme motivierendes Empfinden hervorgerufen werden, dass die Einnahme vorteilhaft insofern erleichtert, als der Patient bzw. Konsument in gewisser Weise zu dieser animiert wird.

Auch ist das Auflöseverhalten der erfindungsgemäßen Darreichungsform, die wie zuvor beschrieben ausgebildet ist, insoweit als vorteilhaft hervorzuhaben, als die kugelförmige Brausetablette weniger als im Stand der Technik bekannte, zylindrische Brausetabletten dazu neigen, am Grund bzw. Boden des Gefäßes, in dem die Tablette aufgelöst wird, anzuhaften, da weniger gerade Fläche für eine Adhäsion am Gefäßboden zur Verfügung steht.

Die erfindungsgemäße Darreichungsform mit der vorgenannten bevorzugten, insbesondere zumindest im Wesentlichen kugelförmigen, Ausgestaltung geht bei direkter oraler Applikation darüber hinaus auch mit einem angenehmen Mundgefühl einher und erlaubt insbesondere ein schnelles und gleichmäßiges Auflösen der der Darreichungsform im Mund, sodass auch die direkte Einnahme der Darreichungsform als angenehm und somit vorteilhaft empfunden und beschrieben werden kann.

Was die diesbezügliche Bereitstellung der erfindungsgemäßen Darreichungsform schließlich anbelangt, so hat es sich im Rahmen der vorliegenden Erfindung als vorteilhaft erwiesen, wenn die Darreichungsform als Brausezubereitung, insbesondere in Form eines losen oder gepressten Pulvers, vorzugsweise in Form einer Brausetablette, durch Homogenisieren, Vermischen und optionales Verpressen der, insbesondere in Form trockener Pulver bereitgestellten, Wirk- und Inhaltsstoffe erhältlich ist bzw. erhalten wird.

Darüber hinaus betrifft die vorliegende Erfindung gemäß dem vorliegenden Aspekt auch folgenden speziellen Ausführungsformen der erfindungsgemäßen Darreichungsform:
So betrifft die vorliegende Erfindung gemäß dem vorliegenden Aspekt auch die erfindungsgemäße Darreichungsform, insbesondere Nahrungsergänzungsmittel- und/oder pharmazeutische Darreichungsform, insbesondere für die orale Applikation, vorzugsweise zur Verwendung bei der prophylaktischen und/oder therapeutischen Behandlung von Infektionen und/oder entzündlichen Erkrankungen des Urogenitaltraktes, insbesondere der Harnwege, und/oder zur unterstützenden präventiven und/oder kurativen Sicherstellung und/oder Förderung der Blasengesundheit, insbesondere wie zuvor beschrieben,
welche in Kombination und in jeweils wirksamen, insbesondere in jeweils Nahrungsergänzungsmittel- und/oder pharmazeutisch wirksamen, Mengen enthält:
   a1) mindestens einen Extrakt von Preiselbeere (*Vaccinium vitis-idaea* L.),
   a2) mindestens einen Extrakt von Großfrüchtiger Moosbeere (*Vaccinium macrocarpon Aiton*) und
   b1) mindestens einen Extrakt von Brennnesselblättern (*Urticae folium*),
wobei die Darreichungsform als Brausezubereitung, insbesondere in Form eines losen oder gepressten Pulvers, vorzugsweise in Form einer Brausetablette, ausgebildet ist;
wobei die Extrakte in der Darreichungsform jeweils in Mengen in einem Bereich von 5 bis 45 Gew.-% bezogen auf die Darreichungsform und berechnet auf Basis der Gesamtzusammensetzung der Extrakte, enthalten sind und/oder
wobei die Extrakte jeweils zusammen mit und/oder in Kombination mit einem Träger formuliert sind und/oder vorliegen, insbesondere wobei die Extrakte den Träger jeweils in Mengen in einem Bereich von 2 bis 70 Gew.-%, bezogen auf die Gesamtzusammensetzung des Extrakts, enthalten und/oder
wobei die Extrakte ein Droge/Extrakt-Verhältnis (DEV) in einem Bereich von 75 : 1 bis 1 :10, jeweils bezogen auf die Gesamtzusammensetzung der Extrakte, aufweisen.

Darüber hinaus betrifft die vorliegende Erfindung gemäß dem vorliegenden Aspekt auch die erfindungsgemäße Darreichungsform, insbesondere Nahrungsergänzungsmittel- und/oder pharmazeutische Darreichungsform, insbesondere für die orale Applikation, vorzugsweise zur Verwendung bei der prophylaktischen und/oder therapeutischen Behandlung von Infektionen und/oder entzündlichen Erkrankungen des Urogenitaltraktes, insbesondere der Harnwege, und/oder zur unterstützenden präventiven und/oder kurativen Sicherstellung und/oder Förderung der Blasengesundheit, insbesondere wie zuvor beschrieben,
die in Kombination und in jeweils wirksamen, insbesondere in jeweils Nahrungsergänzungsmittel- und/oder pharmazeutisch wirksamen, Mengen enthält:
   a1) mindestens einen Extrakt von Preiselbeere (*Vaccinium vitis-idaea* L.),
   a2) mindestens einen Extrakt von Großfrüchtiger Moosbeere (*Vaccinium macrocarpon Aiton*) und
   b1) mindestens einen Extrakt von Brennnesselblättern (*Urticae folium*),
wobei die Darreichungsform als Brausezubereitung, insbesondere in Form eines losen oder gepressten Pulvers, vorzugsweise in Form einer Brausetablette, ausgebildet ist und
wobei die Darreichungsform, einen Proanthocyanidin-Gehalt in einem Bereich von 0,01 bis 5 Gew.-%, insbesondere 0,05 bis 3,5 Gew.-%, vorzugsweise 0,1 bis 3 Gew.- %, bevorzugt 0,5 bis 2,5 Gew.-%, bezogen auf die Darreichungsform, aufweist;
insbesondere wobei die Extrakte in der Darreichungsform jeweils in Mengen in einem Bereich von 5 bis 45 Gew.-% bezogen auf die Darreichungsform und berechnet auf Basis der Gesamtzusammensetzung der Extrakte, enthalten sind und/oder
insbesondere wobei die Extrakte jeweils zusammen mit und/oder in Kombination mit einem Träger formuliert sind und/oder vorliegen, vorzugsweise wobei die Extrakte den Träger jeweils in Mengen in einem Bereich von 2 bis 70 Gew.-%, bezogen auf die Gesamtzusammensetzung des Extrakts, enthalten, und/oder
insbesondere wobei die Extrakte ein Droge/Extrakt-Verhältnis (DEV) in einem Bereich von 75 : 1 bis 1 : 10, jeweils bezogen auf die Gesamtzusammensetzung der Extrakte, aufweisen.

Außerdem betrifft die vorliegende Erfindung gemäß dem vorliegenden Aspekt auch die erfindungsgemäße Darreichungsform, insbesondere Nahrungsergänzungsmittel- und/oder pharmazeutische Darreichungsform, insbesondere für die orale Applikation, vorzugsweise zur Verwendung bei der prophylaktischen und/oder therapeutischen Behandlung von Infektionen und/oder entzündlichen Erkrankungen des Urogenitaltraktes, insbesondere der Harnwege, und/oder zur unterstützenden präventiven und/oder kurativen Sicherstellung und/oder Förderung der Blasengesundheit, insbesondere wie zuvor beschrieben,
welche in Kombination und in jeweils wirksamen, insbesondere in jeweils Nahrungsergänzungsmittel- und/oder pharmazeutisch wirksamen, Mengen enthält:
   a1) mindestens einen Extrakt von Preiselbeere (*Vaccinium vitis-idaea* L.),
   a2) mindestens einen Extrakt von Großfrüchtiger Moosbeere (*Vaccinium macrocarpon Aiton*) und
   b1) mindestens einen Extrakt von Brennnesselblättern (*Urticae folium*),
wobei die Darreichungsform als Brausezubereitung, insbesondere in Form eines losen oder gepressten Pulvers, vorzugsweise in Form einer Brausetablette, ausgebildet ist und
wobei die Extrakte jeweils zusammen mit und/oder in Kombination mit einem Träger formuliert sind und/oder vorliegen, insbesondere wobei der Träger ein wasserlöslicher oder wasserdispergierbarer, insbesondere Kohlenhydrat-basierter und/oder -haltiger, vorzugsweise Oligo- und/oder Polysaccharid-basierter und/oder - haltiger, Träger ist, bevorzugt Maltodextrin ist;
insbesondere wobei a1) der Extrakt von Preiselbeere (*Vaccinium vitis-idaea* L.) den Träger in Mengen in einem Bereich von 2 bis 10 Gew.-%, bezogen auf die Gesamtzusammensetzung des Extrakts, enthält und/oder
insbesondere wobei a2) der Extrakt von Großfrüchtiger Moosbeere (*Vaccinium macrocarpon Aiton*) den Träger in Mengen in einem Bereich von 20 bis 70 Gew.-%, bezogen auf die Gesamtzusammensetzung des Extrakts, enthält und/oder
insbesondere wobei b1) der mindestens eine Extrakt von Brennnesselblättern (*Urticae folium*), den Träger in Mengen in einem Bereich von 20 bis 70 Gew.-%, bezogen auf die Gesamtzusammensetzung des Extrakts, enthält, und/oder
insbesondere wobei a1) der Extrakt von Preiselbeere (*Vaccinium vitis-idaea* L.) in der Darreichungsform in Mengen in einem Bereich von 1 bis 20 Gew.-%, bezogen auf die Darreichungsform und berechnet auf Basis der Gesamtzusammensetzung des Extrakts, enthalten ist und/oder ein Droge/Extrakt-Verhältnis (DEV) in einem Bereich von 75: 1 bis 35: 1, bezogen auf die Gesamtzusammensetzung des Extrakts, aufweist und/oder
insbesondere wobei a2) der Extrakt von Großfrüchtiger Moosbeere (*Vaccinium macrocarpon Aiton*) in der Darreichungsform in Mengen in einem Bereich von 1 bis 20 Gew.-%, bezogen auf die Darreichungsform und berechnet auf Basis der Gesamtzusammensetzung des Extrakts, enthalten ist und/oder ein Droge/Extrakt-Verhältnis (DEV) in einem Bereich von 20 : 1 bis 1 : 10, bezogen auf die Gesamtzusammensetzung des Extrakts, aufweist und/oder
insbesondere wobei b1) der Extrakt von Brennnesselblättern (*Urticae folium*) in der Darreichungsform in Mengen in einem Bereich von 1 bis 20 Gew.-%, bezogen auf die Darreichungsform und berechnet auf Basis der Gesamtzusammensetzung des Extrakts, enthalten ist und/oder ein Droge/Extrakt-Verhältnis (DEV) in einem Bereich von 20 : 1 bis 1 : 10, bezogen auf die Gesamtzusammensetzung des Extrakts, aufweist.

Weiterhin betrifft die vorliegende Erfindung gemäß dem vorliegenden Aspekt auch die erfindungsgemäße Darreichungsform, insbesondere Nahrungsergänzungsmittel- und/oder pharmazeutische Darreichungsform, insbesondere für die orale Applikation, vorzugsweise zur Verwendung bei der prophylaktischen und/oder therapeutischen Behandlung von Infektionen und/oder entzündlichen Erkrankungen des Urogenitaltraktes, insbesondere der Harnwege, und/oder zur unterstützenden präventiven und/oder kurativen Sicherstellung und/oder Förderung der Blasengesundheit, insbesondere wie zuvor beschrieben,
die in Kombination und in jeweils wirksamen, insbesondere in jeweils Nahrungsergänzungsmittel- und/oder pharmazeutisch wirksamen, Mengen enthält:
   a1) mindestens einen Extrakt von Preiselbeere (*Vaccinium vitis-idaea* L.),
   a2) mindestens einen Extrakt von Großfrüchtiger Moosbeere (*Vaccinium macrocarpon Aiton*) und
   b1) mindestens einen Extrakt von Brennnesselblättern (*Urticae folium*),
wobei die Darreichungsform als Brausezubereitung, insbesondere in Form eines losen oder gepressten Pulvers, vorzugsweise in Form einer Brausetablette, ausgebildet ist und
wobei die Extrakte jeweils zusammen mit und/oder in Kombination mit einem Träger formuliert sind und/oder vorliegen, insbesondere wobei der Träger ein wasserlöslicher oder wasserdispergierbarer, insbesondere Kohlenhydrat-basierter und/oder -haltiger, vorzugsweise Oligo- und/oder Polysaccharid-basierter und/oder - haltiger, Träger ist, bevorzugt Maltodextrin ist;
wobei a1) der Extrakt von Preiselbeere (*Vaccinium vitis-idaea* L.) den Träger in Mengen in einem Bereich von 2 bis 10 Gew.-%, bezogen auf die Gesamtzusammensetzung des Extrakts, enthält und
wobei a2) der Extrakt von Großfrüchtiger Moosbeere (*Vaccinium macrocarpon Aiton*) den Träger in Mengen in einem Bereich von 20 bis 70 Gew.-%, bezogen auf die Gesamtzusammensetzung des Extrakts, enthält und
wobei b1) der mindestens eine Extrakt von Brennnesselblättern (*Urticae folium*), den Träger in Mengen in einem Bereich von 20 bis 70 Gew.-%, bezogen auf die Gesamtzusammensetzung des Extrakts, enthält, und
wobei a1) der Extrakt von Preiselbeere (*Vaccinium vitis-idaea* L.) in der Darreichungsform in Mengen in einem Bereich von 1 bis 20 Gew.-%, bezogen auf die Darreichungsform und berechnet auf Basis der Gesamtzusammensetzung des Extrakts, enthalten ist und ein Droge/Extrakt-Verhältnis (DEV) in einem Bereich von 75 : 1 bis 35 : 1, bezogen auf die Gesamtzusammensetzung des Extrakts, aufweist und
wobei a2) der Extrakt von Großfrüchtiger Moosbeere (*Vaccinium macrocarpon Aiton*) in der Darreichungsform in Mengen in einem Bereich von 1 bis 20 Gew.-%, bezogen
auf die Darreichungsform und berechnet auf Basis der Gesamtzusammensetzung des Extrakts, enthalten ist und ein Droge/Extrakt-Verhältnis (DEV) in einem Bereich von 20 : 1 bis 1 : 10, bezogen auf die Gesamtzusammensetzung des Extrakts, aufweist und
wobei b1) der Extrakt von Brennnesselblättern (*Urticae folium*) in der Darreichungsform in Mengen in einem Bereich von 1 bis 20 Gew.-%, bezogen auf die Darreichungsform und berechnet auf Basis der Gesamtzusammensetzung des Extrakts, enthalten ist und ein Droge/Extrakt-Verhältnis (DEV) in einem Bereich von 20 : 1 bis 1 : 10, bezogen auf die Gesamtzusammensetzung des Extrakts, aufweist, und
wobei die Darreichungsform, einen Proanthocyanidin-Gehalt in einem Bereich von 0,01 bis 5 Gew.-%, insbesondere 0,05 bis 3,5 Gew.-%, vorzugsweise 0,1 bis 3 Gew.- %, bevorzugt 0,5 bis 2,5 Gew.-%, bezogen auf die Darreichungsform, aufweist.

Schließlich betrifft die vorliegende Erfindung gemäß dem vorliegenden Aspekt auch die erfindungsgemäße Darreichungsform, insbesondere Nahrungsergänzungsmittel- und/oder pharmazeutische Darreichungsform, insbesondere für die orale Applikation, vorzugsweise zur Verwendung bei der prophylaktischen und/oder therapeutischen Behandlung von Infektionen und/oder entzündlichen Erkrankungen des Urogenitaltraktes, insbesondere der Harnwege, und/oder zur unterstützenden präventiven und/oder kurativen Sicherstellung und/oder Förderung der Blasengesundheit, insbesondere wie zuvor beschrieben,
welche in Kombination und in jeweils wirksamen, insbesondere in jeweils Nahrungsergänzungsmittel- und/oder pharmazeutisch wirksamen, Mengen enthält:
   a1) mindestens einen Extrakt von Preiselbeere (*Vaccinium vitis-idaea* L.),
   a2) mindestens einen Extrakt von Großfrüchtiger Moosbeere (*Vaccinium macrocarpon Aiton*) und
   b1) mindestens einen Extrakt von Brennnesselblättern (*Urticae folium*).
wobei die Darreichungsform als Brausezubereitung, insbesondere in Form eines losen oder gepressten Pulvers, vorzugsweise in Form einer Brausetablette, ausgebildet ist und
wobei die Extrakte in der Darreichungsform jeweils in Mengen in einem Bereich von 5 bis 45 Gew.-% bezogen auf die Darreichungsform und berechnet auf Basis der Gesamtzusammensetzung der Extrakte, enthalten sind und
wobei die Extrakte jeweils zusammen mit und/oder in Kombination mit einem Träger formuliert sind und/oder vorliegen, insbesondere wobei die Extrakte den Träger jeweils in Mengen in einem Bereich von 2 bis 70 Gew.-%, bezogen auf die Gesamtzusammensetzung des Extrakts, enthalten und/oder insbesondere wobei der Träger ein wasserlöslicher oder wasserdispergierbarer, insbesondere Kohlenhydrat-basierter und/oder -haltiger, vorzugsweise Oligo- und/oder Polysaccharid-basierter und/oder -haltiger, Träger ist, bevorzugt Maltodextrin ist, und
wobei die Extrakte jeweils ein Droge/Extrakt-Verhältnis (DEV) in einem Bereich von 75 : 1 bis 1 : 10, jeweils bezogen auf die Gesamtzusammensetzung der Extrakte, aufweisen und
wobei die Darreichungsform wobei die Darreichungsform als weitere Inhaltsstoffe Vitamin C und Trimagnesiumdicitrat umfasst, wobei die Darreichungsform Vitamin C in Mengen in einem Bereich von 0,1 bis 5 Gew.-%, und Trimagnesiumdicitrat in Mengen in einem Bereich von 25 bis 40 Gew.-%, jeweils bezogen auf die Darreichungsform, enthält

Für weitergehende Einzelheiten zu dem vorstehend beschriebenen Erfindungsaspekt kann zudem Bezug genommen werden auf die Ausführungen zu den weiteren Erfindungsaspekten, wobei diese Ausführungen gleichermaßen für den vorliegenden Erfindungsaspekt entsprechend gelten.

Zudem ist Gegenstand der vorliegenden Erfindung - gemäß einem **zweiten** Aspekt der vorliegenden Erfindung - ist zudem eine Darreichungsform nach der vorliegenden Erfindung zur Verwendung bei der prophylaktischen und/oder therapeutischen Behandlung von Infektionen und/oder entzündlichen Erkrankungen des Urogenitaltraktes, insbesondere der Harnwege, und/oder zur prophylaktischen oder therapeutischen Behandlung von insbesondere chronisch rezidivierenden Harnwegsinfekten und/oder bei der, insbesondere ergänzenden und/oder unterstützenden, präventiven und/oder kurativen Versorgung mit Nährstoffen und aktiven Inhaltsstoffen zur Sicherstellung und/oder Förderung der Blasengesundheit.

Für weitergehende Einzelheiten zu diesem Erfindungsaspekt, insbesondere auch zu den assoziierten Vorteilen und besonderen technischen Wirkungen bzw. Effekten, kann Bezug genommen werden auf die Ausführungen zu den weiteren Erfindungsaspekten, wobei diese Ausführungen gleichermaßen für den vorliegenden Erfindungsaspekt entsprechend gelten.

Wiederum weiterer Gegenstand der vorliegenden Erfindung - gemäß einem **dritten** Aspekt der vorliegenden Erfindung - ist die Verwendung einer Darreichungsform nach der vorliegenden Erfindung im Nahrungsergänzungsbereich, insbesondere zur nicht-therapeutischen und/oder nicht-medizinischen Vorbeugung von Infektionen und/oder entzündlichen Erkrankungen des Urogenitaltraktes, insbesondere der Harnwege, und/oder bei der, insbesondere ergänzenden, präventiven und/oder kurativen Versorgung mit Nährstoffen und aktiven Inhaltsstoffen zur Sicherstellung und/oder Förderung der Blasengesundheit.

Für weitergehende Einzelheiten zu diesem Erfindungsaspekt, insbesondere auch zu den assoziierten Vorteilen und besonderen technischen Wirkungen bzw. Effekten, kann Bezug genommen werden auf die Ausführungen zu den weiteren Erfindungsaspekten, wobei diese Ausführungen gleichermaßen für den vorliegenden Erfindungsaspekt entsprechend gelten.

Weiterer Gegenstand der vorliegenden Erfindung - gemäß einem **vierten** Aspekt der vorliegenden Erfindung - ist eine Brausezubereitung, insbesondere für die orale Applikation, insbesondere in Form eines losen oder gepressten Pulvers, vorzugsweise Brausetablette, bestehend aus einer Darreichungsform nach der vorliegenden Erfindung, insbesondere zur Verwendung bei der prophylaktischen und/oder therapeutischen Behandlung von Infektionen und/oder entzündlichen Erkrankungen des Urogenitaltraktes, insbesondere der Harnwege, und/oder zur prophylaktischen oder therapeutischen Behandlung von insbesondere chronisch rezidivierenden Harnwegsinfekten und/oder bei der, insbesondere ergänzenden, präventiven und/oder kurativen Versorgung mit Nährstoffen und aktiven Inhaltsstoffen zur Sicherstellung und/oder Förderung der Blasengesundheit.

Für weitergehende Einzelheiten zu diesem Erfindungsaspekt, insbesondere auch zu den assoziierten Vorteilen und besonderen technischen Wirkungen bzw. Effekten, kann Bezug genommen werden auf die Ausführungen zu den weiteren Erfindungsaspekten, wobei diese Ausführungen gleichermaßen für den vorliegenden Erfindungsaspekt entsprechend gelten.

Wiederum weiterer Gegenstand der vorliegenden Erfindung - gemäß einem **fünften** Aspekt der vorliegenden Erfindung - ist Verwendung einer erfindungsgemäßen Brausezubereitung im Nahrungsergänzungsbereich, insbesondere zur nicht-therapeutischen und/oder nicht-medizinischen Vorbeugung von Infektionen und/oder entzündlichen Erkrankungen des Urogenitaltraktes, insbesondere der Harnwege, und/oder bei der, insbesondere ergänzenden, präventiven und/oder kurativen Versorgung mit Nährstoffen und aktiven Inhaltsstoffen zur Sicherstellung und/oder Förderung der Blasengesundheit.

Für weitergehende Einzelheiten zu diesem Erfindungsaspekt, insbesondere auch zu den assoziierten Vorteilen und besonderen technischen Wirkungen bzw. Effekten, kann Bezug genommen werden auf die Ausführungen zu den weiteren Erfindungsaspekten, wobei diese Ausführungen gleichermaßen für den vorliegenden Erfindungsaspekt entsprechend gelten.

Wiederum weiterer Gegenstand der vorliegenden Erfindung - gemäß einem **sechsten** Aspekt der vorliegenden Erfindung - ist eine Brausetablette (1), insbesondere umfassend eine Nahrungsergänzungsmittel- und/oder pharmazeutische Darreichungsform, insbesondere für die orale Applikation, vorzugsweise zur Verwendung bei der prophylaktischen und/oder therapeutischen Behandlung von Infektionen und/oder entzündlichen Erkrankungen des Urogenitaltraktes, insbesondere der Harnwege, und/oder zur unterstützenden präventiven und/oder kurativen Sicherstellung und/oder Förderung der Blasengesundheit,
wobei die Brausetablette zumindest im Wesentlichen in Form einer Kugel, insbesondere in Form einer insbesondere oval verzerrten Kugel oder eines Ellipsoids, mit mittig umlaufendem Steg (3), ausgebildet ist.

Vorzugsweise ist es dabei vorgesehen, dass der mittig umlaufende Steg (3) einen zumindest im Wesentlichen zylindrischen Abschnitt ausbildet, vorzugsweise sodass eine Braustablette in Form zweier zusammengesetzter, durch einen insbesondere zylindrisch geformten, mittig umlaufenden Steg (3) verbundener, Halbkugeln (2a, 2b) ausgebildet ist.

Die besondere Form der erfindungsgemäßen Brausetablette gestattet insbesondere eine besonders gleichmäßige Verteilung der Inhalts- bzw. Wirkstoffe in der Tablette auf Grund des bei der Tablettenform besonders vorteilhaften Volumenverhältnis gegenüber anderen relevanten Parametern wie dem Gewicht.

Darüber hinaus kann auf Grund der kugelförmigen Ausgestaltung der erfindungsgemäßen Brausetablette ein beschleunigter Zerfall bzw. eine kurze Zerfallszeit erreicht werden. Die im Verhältnis zum Volumen große Oberfläche der Kugel gestattet insbesondere eine stabilere, großflächigere und damit insgesamt schnellere Freisetzung von Kohlendioxid aus der Brausetablette, wodurch gleichermaßen vorteilhaft auch der Zerfall der Tablette beschleunigt abläuft.

Die kompakte sowie runde Form der Tablette ermöglicht außerdem vorteilhaft auch, dass die erfindungsgemäße Tablette eine geringere Bruchneigung aufweist bzw. eine verbesserte Bruchstabilität erhält, insbesondere im Vergleich zu herkömmlichen, in Form von flachen Zylindern ausgebildeten Tabletten.

Die Form der erfindungsgemäßen Brausetablette ermöglicht überdies vorteilhaft das Einstellen besonders glatter Oberflächen, was gleichzeitig auch vorteilhaft dazu beiträgt, dass die Tablette in geringerem Maße zur Aufnahme von Feuchtigkeit aus der Luft neigt. Die Stabilität und Haltbarkeit der erfindungsgemäßen Tablette ist entsprechend gegenüber herkömmlichen Tabletten erhöht.

Durch den Steg, welchen die erfindungsgemäße Tablette aufweist, kann außerdem der Herstellungsprozess vorteilhaft stabilisiert werden, indem die Kompaktierung der Tablette bzw. der Pressvorgang effizienter und sicherer gestaltet werden können, insbesondere da die Tablette auf Grund des Stegs nicht einfach wegrollen kann. Dies ist gleichermaßen vorteilhaft für die Anwendung und Handhabung der erfindungsgemäßen Brausetablette bzw. bei der Applikation.

Zudem ist die erfindungsgemäße Brausetablette aufgrund ihrer Form und insbesondere auch ihrer Abmaße vorteilhaft so ausgebildet, dass sie durch die Öffnungen herkömmlicher Trinkflaschen passt und entsprechend unkompliziert in einer Trinkflasche aufgelöst werden kann, so dass vorteilhaft ein Getränk auf Basis der erfindungsgemäßen Brausetablette in einem wiederverschließbaren Gefäß zubereitet werden kann.

Bevorzugt enthält die Brausetablette eine Darreichungsform und/oder eine Brausezubereitung je nach der vorliegenden Erfindung.

Es betrifft die vorliegende Erfindung gemäß dem vorliegenden Aspekt auch die erfindungsgemäße Brausetablette (1), insbesondere Brausetablette wie zuvor beschrieben, insbesondere umfassend eine Nahrungsergänzungsmittel- und/oder pharmazeutische Darreichungsform, insbesondere für die orale Applikation, vorzugsweise zur Verwendung bei der prophylaktischen und/oder therapeutischen Behandlung von Infektionen und/oder entzündlichen Erkrankungen des Urogenitaltraktes, insbesondere der Harnwege, und/oder zur unterstützenden präventiven und/oder kurativen Sicherstellung und/oder Förderung der Blasengesundheit, insbesondere umfassend eine Darreichungsform und/oder eine Brausezubereitung nach der vorliegenden Erfindung,
wobei die Brausetablette zumindest im Wesentlichen in Form einer Kugel, insbesondere in Form einer insbesondere oval verzerrten Kugel oder eines Ellipsoids, mit mittig umlaufendem Steg (3), ausgebildet ist,
wobei der mittig umlaufende Steg (3) einen zumindest im Wesentlichen zylindrischen Abschnitt ausbildet, vorzugsweise sodass eine Braustablette in Form zweier zusammengesetzter, durch einen insbesondere zylindrisch geformten, mittig umlaufenden Steg (3) verbundener, Halbkugeln (2a, 2b) ausgebildet ist.

Für weitergehende Einzelheiten zu diesem Erfindungsaspekt, insbesondere auch zu den assoziierten Vorteilen und besonderen technischen Wirkungen bzw. Effekten, kann Bezug genommen werden auf die Ausführungen zu den weiteren Erfindungsaspekten, wobei diese Ausführungen gleichermaßen für den vorliegenden Erfindungsaspekt entsprechend gelten.

Schließlich Gegenstand der vorliegenden Erfindung - gemäß einem **siebten** Aspekt der vorliegenden Erfindung - ist eine Verpackungseinheit, enthaltend mindestens eine Darreichungsform, vorzugsweise eine Vielzahl von Darreichungsformen, nach der vorliegenden Erfindung und/oder enthaltend mindestens eine Brausezubereitung, vorzugsweise eine Vielzahl von Brausezubereitungen, nach der vorliegenden Erfindung und/oder enthaltend mindestens eine Brausetablette, vorzugsweise eine Vielzahl von Brausetabletten, nach der vorliegenden Erfindung.

Bevorzugt ist es dabei, wenn die Verpackungseinheit eine Blisterverpackung ist und/oder als Blisterverpackung ausgebildet ist, vorzugsweise als Durchdrückblisterverpackung.

Diesbezüglich hat es sich bewährt, wenn die Blisterverpackung eine insbesondere aus Kunststoff gebildete Aufnahmeform mit einer Vielzahl von Mulden für die Aufnahme der Darreichungsform(en) und/oder der Brausezubereitung(en) und/oder der Brausetablette(n) sowie eine mit der Aufnahmeform abdichtend verbundene, die Mulden verschließende Deckfolie, insbesondere Blisterfolie, bevorzugt Aluminiumfolie, umfasst.

Für weitergehende Einzelheiten zu diesem Erfindungsaspekt, insbesondere auch zu den assoziierten Vorteilen und besonderen technischen Wirkungen bzw. Effekten, kann Bezug genommen werden auf die Ausführungen zu den weiteren Erfindungsaspekten, wobei diese Ausführungen gleichermaßen für den vorliegenden Erfindungsaspekt entsprechend gelten.

Die vorliegende Erfindung sowohl gemäß dem ersten Aspekt der vorliegenden Erfindung als auch gemäß sämtlichen übrigen Aspekten der vorliegenden Erfindung ist insgesamt mit einer Vielzahl von Vorteilen und Besonderheiten verbunden, welche das erfindungsgemäße Anwendungs- bzw. Therapiekonzept einzigartig und hocheffizient machen.

Im Folgenden wird die vorliegende Erfindung anhand von bevorzugten Ausführungsbeispielen und anhand von eine bevorzugte Ausführungsform darstellenden Zeichnungen bzw. Figurendarstellungen näher erläutert. Im Zusammenhang mit der Erläuterung dieser bevorzugten Ausführungsbeispiele bzw. Ausführungsformen der vorliegenden Erfindung, welche jedoch in Bezug auf die vorliegende Erfindung keinesfalls beschränkend sind, werden auch weitergehende Vorteile, Eigenschaften, Aspekte und Merkmale der vorliegenden Erfindung aufgezeigt.

In den Figurendarstellungen zeigt:
- Fig. 1: eine schematische Darstellung mit einer perspektivischen Ansicht auf die erfindungsgemäße Darreichungsform 1 in Form einer Brausezubereitung, ausgebildet als Brausetablette,
- Fig. 2A: eine weitere schematische Darstellung mit einer Seitenansicht auf die erfindungsgemäße Darreichungsform 1 in Form einer Brausezubereitung, ausgebildet als Brausetablette,
- Fig. 2B: eine weitere schematische Darstellung mit einer Draufsicht auf die erfindungsgemäße Darreichungsform 1 in Form einer Brausezubereitung, ausgebildet als Brausetablette.

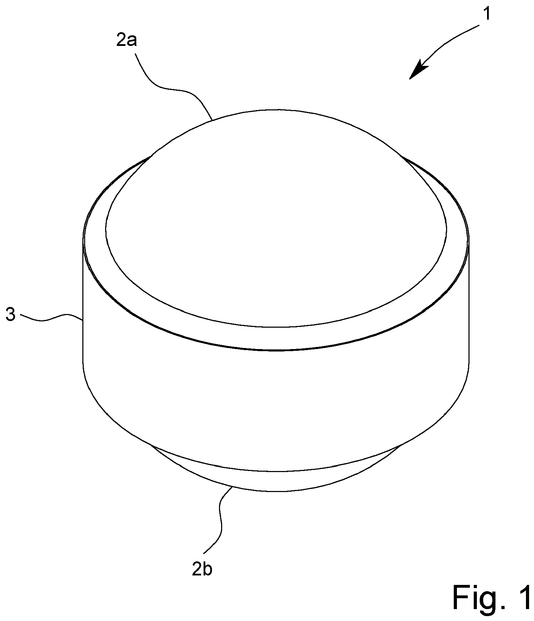

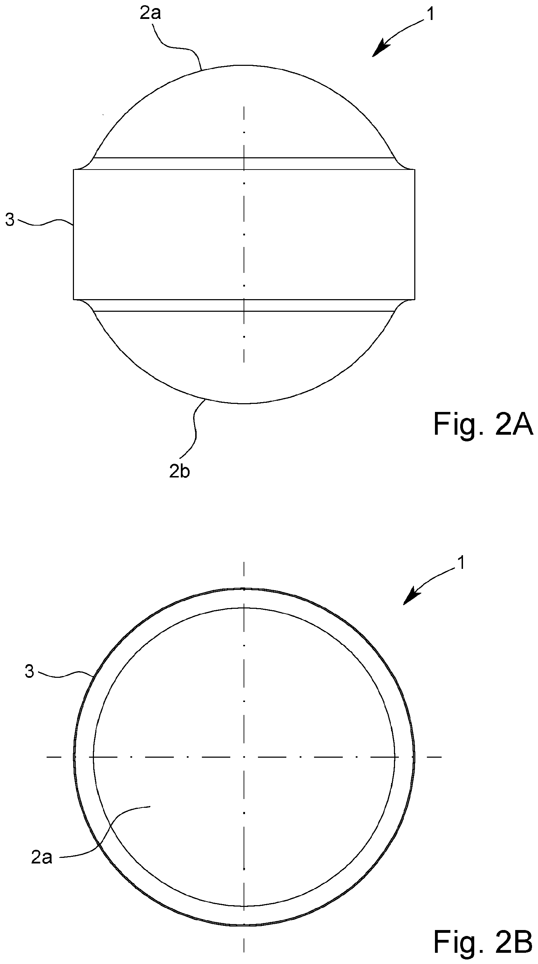

Insgesamt verdeutlichen die Figurendarstellungen gemäß Fig. 1, 2A und 2B die erfindungsgemäße Konzeption mit der Bereitstellung einer speziellen Darreichungsform 1, insbesondere Nahrungsergänzungsmittel- und/oder pharmazeutische Darreichungsform, insbesondere für die orale Applikation, vorzugsweise zur Verwendung bei der prophylaktischen und/oder therapeutischen Behandlung von Infektionen und/oder entzündlichen Erkrankungen des Urogenitaltraktes, insbesondere der Harnwege, und/oder zur unterstützenden präventiven und/oder kurativen Sicherstellung und/oder Förderung der Blasengesundheit,
wobei die Darreichungsform als Brausezubereitung, insbesondere in Form eines losen oder gepressten Pulvers, vorzugsweise in Form einer Brausetablette, ausgebildet ist,
wobei die Darreichungsform eine Kombination von mindestens einer Proanthocyanidine-enthaltenden Wirkstoffkomponente einerseits und mindestens einer diuretisch-wirksamen und/oder entzündungshemmenden Wirkstoffkomponente andererseits umfasst, und
wobei die Kombination
   a) mindestens einen Extrakt, insbesondere mindestens zwei Extrakte, von Pflanzen aus der Familie der Heidekrautgewächse (Ericaceae), insbesondere der Gattung Heidelbeere (Vaccinium), einerseits und
   b) mindestens einen Extrakt von Pflanzen aus der Familie der Brennnesselgewächse (Urticaceae) andererseits enthält.

Die in Figur 1 schematisch gezeigte bevorzugte Ausführung der erfindungsgemäßen Darreichungsform 1 als Brausezubereitung in Form einer Brausetablette ist zumindest im Wesentlichen kugelförmig, insbesondere in Form einer insbesondere oval verzerrten Kugel oder eines Ellipsoids jeweils mit mittig umlaufendem Steg 3, ausgebildet.

Bevorzugt bildet der mittig umlaufende Steg 3 dabei einen zumindest im Wesentlichen zylindrischen Abschnitt aus, vorzugsweise so dass die Braustablette in Form zweier zusammengesetzter, durch einen insbesondere zylindrisch geformten, mittig umlaufenden Steg 3 verbundener, Halbkugeln 2a,b ausgebildet ist.

In anderen Worten weist die Darreichungsform 1 im Rahmen dieser bevorzugten Ausgestaltung der Darreichungsform als Brausetablette eine Form auf, die eine obere Halbkugel 2a und eine untere Halbkugel 2b umfasst, wobei die Halbkugeln 2a und 2b durch einen mittig umlaufenden, insbesondere zylindrisch geformten, Steg 3 miteinander verbunden sind. Insgesamt ergibt sich so eine Darreichungsform 1 in Form einer insbesondere oval verzerrten Kugel oder eines Ellipsoids. Für diese bevorzugte Ausführung wird vorteilhaft ein nahezu optimales Oberfläche-/VolumenVerhältnis erreicht, sodass eine im Hinblick auf den Umfang bzw. die Größe der Dosier- bzw. Packungseinheit möglichst effiziente, kleinvolumige Formulierung einer Dosis der Darreichungsform möglich ist.

Darüber hinaus wird für diese Ausgestaltung der Brausetablette ebenfalls ein sehr gutes sowie auch charakteristisches Löslichkeitsverhalten der Darreichungsform in wässriger Lösung beobachtet. Es wird vorteilhaft sowohl ein gleichmäßiges und zügiges sowie vollständiges und rückstandsfreies Auflösen der Brausetablette erreicht. Auf Grund der Ausgestaltung der in Form einer insbesondere oval verzerrten Kugel oder eines Ellipsoids ausgebildeten Brausetablette, die einen mittig umlaufenden Steg 3 aufweist, insbesondere wobei der mittig umlaufende Steg 3 einen zumindest im Wesentlichen zylindrischen Abschnitt ausbildet, sodass eine Braustablette in Form zweier zusammengesetzter, durch einen insbesondere zylindrisch geformten, mittig umlaufenden Steg 3 verbundener, Halbkugeln 2a,b ausgebildet ist, schwimmt die Darreichungsform beim Auflösen in Wasser auf der Wasseroberfläche auf und führt eine als "tanzend" anmutende Taumelbewegung auf der Wasseroberfläche aus. Vorteilhaft kann so verfolgt werden, ob bzw. dass sich die Tablette rückstandsfrei sowie vollständig auflöst. Außerdem kann beim Patienten bzw. Konsumenten ein visuell anregendes sowie auch zur Einnahme motivierendes Empfinden hervorgerufen werden, das die Einnahme vorteilhaft insofern erleichtert, als der Patient bzw. Konsument in gewisser Weise zu dieser animiert wird.

Bei direkter oraler Applikation zeichnet sich die Darreichungsform 1 darüber hinaus durch ein angenehmes Mundgefühl aus und erlaubt insbesondere ein schnelles und gleichmäßiges Auflösen der der Darreichungsform im Mund, sodass auch die direkte Einnahme der Darreichungsform als angenehm und somit vorteilhaft empfunden und beschrieben werden kann.

Was die diesbezügliche Bereitstellung der erfindungsgemäßen Darreichungsform 1 anbelangt, so hat es sich bewährt, wenn die Darreichungsform als Brausezubereitung 1 durch Homogenisieren, Vermischen und Verpressen der, insbesondere in Form trockener Pulver bereitgestellten, Wirk- und Inhaltsstoffe erhältlich ist bzw. erhalten wird.

Für weitergehende Einzelheiten zu den in den Figurendarstellungen dargestellten Ausführungsformen und Ausführungsbeispielen kann zur Vermeidung unnötiger Wiederholungen auch auf die obigen Ausführungen sowie auf die nachfolgenden ergänzenden Ausführungen zu den Ausführungsbeispielen verwiesen werden, welche in Bezug auf die Figurendarstellungen entsprechend gelten.

Weitere Ausgestaltungen, Abwandlungen und Variationen sowie Vorteile der vorliegenden Erfindung sind für den Fachmann beim Lesen der Beschreibung ohne Weiteres erkennbar und realisierbar, ohne dass er dabei den Rahmen der vorliegenden Erfindung verlässt.

Die folgenden Ausführungsbeispiele dienen lediglich der Veranschaulichung der vorliegenden Erfindung, ohne die vorliegende Erfindung jedoch hierauf zu beschränken.

### Ausführungsbeispiele

### 1. Herstellung einer erfindungsgemäßen Darreichungsform als Brausezubereitung

Vorliegend werden erfindungsgemäße Darreichungsformen in Form von Brausezubereitungen, ausgebildet als Brausepulver sowie als Brausetablette, auf Basis der nachfolgend in Tabelle 1 angegebenen zwei Grundrezepturen hergestellt.

**Tabelle 1:**

| **Wirk- bzw. Inhaltsstoffe für Grundrezeptur** | | | **Grundrezeptur 1** | | **Grundrezeptur 2** | |
|---|---|---|---|---|---|---|
| | | | [Gew.-%] | [mg] | [Gew.-%] | [mg] |
| | | | | | | |
| Wirkstoffe bzw. aktive Inhaltsstoffe | Preiselbeer-Extrakt: | | 6,56 | 100,0 | 6,56 | 100,0 |
| | DEV: 50 : 1, min. 25 % Proanthocyanidine, aus: 95 % Extrakt aus Preiselbeere (*Vaccinium vitis-idaea L.*), 5 % Träger (Maltodextrin) | | | | | |
| | Moosbeer-Extrakt: | | 6,56 | 100,0 | 6,56 | 100,0 |
| | DEV: 4 - 6 : 1, aus: 50 - 70 % Trockenextrakt aus großfrüchtiger Moosbeere (*Vaccinium macrocarpon Aiton*), 30 - 50 % Träger (Maltodextrin) | | | | | |
| | Brennnesselblätter-Extrakt: | | 6,56 | 100,0 | 6,56 | 100,0 |
| | DEV: 4 : 1, aus 50 - 70 % Trockenextrakt aus Brennnesselblättern (*Urticae folium*), 30 - 50 % Träger (Maltodextrin) | | | | | |
| zusätzl. aktive Inhaltsstoffe | L-Ascorbinsäure (Vitamin C) | | 1,14 | 17,4 | 1,35 | 20,6 |
| | 97,0 % DC, aus: 97,0 % L-Ascorbinsäure, 3,0 % Hydroxypropylmethylcellulose | | | | | |
| | Tri-Magnesiumdicitrat, wasserfrei, 15,0 - 16,4 % Magnesium | | 32,78 | 500,0 | 32,78 | 500,0 |
| weitere Inhalts- und Hilfsstoffe | Sucralose (E955) | | 0,98 | 15,0 | 0,98 | 15,0 |
| | Himbeer-Aroma | | 0,79 | 12,0 | 0,79 | 12,0 |
| | Natriumbicarbonat (E500(ii)) | | 11,80 | 180,0 | 10,75 | 164,0 |
| | Citronensäure (wasserfrei, E330) | | 24,63 | 375,6 | 24,59 | 375,0 |
| | Isomalt (E953) | | 5,25 | 80,0 | 4,16 | 63,4 |
| | Hydroxypropylcellulose | | 2,95 | 45,0 | 2,95 | 45,0 |
| | Croscarmellose Natrium 4 % | | | | 1,97 | 30,0 |
| **Gesamtzusammensetzung/-menge:** | | | **ad 100,00** | **ad 1.525,0** | **ad 100,00** | **ad 1.525,0** |

Dabei wird derart vorgegangen, dass die insbesondere trockenen sowie pulverförmigen Inhaltsstoffe der Darreichungsform innig bzw. homogen miteinander vermischt werden, ggf. unter Einstellung einer geeigneten Restfeuchte, insbesondere für die Herstellung von Brausetabletten.

Für die Darreichungsform als Brausepulver wird die aus dem Mischvorgang erhaltene Zusammensetzung in Mengen, die der in Tabelle 1 angegebenen Packungs- bzw. Dosiereinheit entsprechen, in feuchtigkeitsgeschützte Verpackungseinheiten abgefüllt.

Für die Darreichungsform als Brausetablette wird eine Mengen der Mischung, die der in Tabelle 1 angegebenen Packungs- bzw. Dosiereinheit entspricht, in eine diesbezügliche Tabletten-Presskammer (Matrize) eingebracht, wobei nachfolgend unter Einwirkung einer Presskraft (Kompressionskraft) von etwa 36 Kilonewton (kN) ein Pressling erhalten wird. Anschließend erfolgt der Auswurf des Presslings aus der Presskammer bzw. Matrize unter Erhalt der erfindungsgemäßen Darreichungsform als Brausetablette. Die so erhaltene Brausetablette weist eine Härte von ca. 38 N auf, bestimmt gemäß *Ph. Eur.* 2.9.8. (*European Pharmacopoeia 9.0*), zeichnet sich also durch eine vergleichsweise hohe Härte aus.

Bevorzugt wird die Brausetablette dabei in eine Kugelform bzw. eine im Wesentlichen, ggf. oval verzerrte, kugelförmige Gestalt gebracht, insbesondere wobei eine obere und eine untere Halbkugel ausgebildet werden, die durch einen mittig umlaufenden Steg verbunden sind, wie in den Figuren 1, 2A und 2B schematisch dargestellt. Die hergestellten Brausetabletten weisen dabei einen Durchmesser in einem Bereich von 12,9 bis 13,3 mm und eine Dicke in einem Bereich von 12,7 bis 13,3 mm auf, zeichnen sich also durch eine Größe aus, die sowohl für die orale Einnahme hervorragend geeignet ist als auch vorteilhaft kompakt für die Verwendung als Brausetablette ist.

Die hergestellten Brausetabletten werden bevorzugt in Verpackungseinheiten in Form von Durchdrück- bzw. Sichtverpackungen einzeln entnehmbar verpackt. So kann vorteilhaft eine hygienische Einzelentnahme der Tabletten gewährleistet werden und sind die Tabletten vor Feuchtigkeit und Schmutz geschützt.

### 2. Untersuchung der Anwendungseigenschaften der Brausezubereitungen der erfindungsgemäßen Darreichungsform

### a. Brausepulverzubereitung

Die Brausepulverzubereitung auf Basis beider Grundrezepturen 1 und 2 ist so formuliert, dass diese direkt als Pulver sowie in Wasser aufgelöst als Getränk eingenommen werden kann.

Die Einnahme als Pulver bietet sich in akuten Fällen, bspw. bei der therapeutischen Behandlung von Infektionen des Urogenitaltraktes (z.B. Zystitis), sowie, hinsichtlich der Verwendung als Nahrungsergänzungsmittel, bei einem erhöhten Nährstoffbedarf, bspw. nach sportlicher Belastung oder aufgrund bspw. alterstypischem Nährstoffmangel, an. Als Pulver kann die erfindungsgemäß Darreichungsform gut geschluckt werden. Außerdem ist auch eine direkte und erhöhte Absorption der Wirk- und Nährstoffe bereits über die Mundschleimhaut möglich.

Die Einnahme als Getränk bietet sich insbesondere für die, vorzugsweise regelmäßige, Verwendung als Nahrungsergänzungsmittel sowie für die prophylaktische und auch therapeutische Behandlung von Infektionen des Urogenitaltraktes an. Durch die Sicherstellung der Zufuhr von zusätzlicher Flüssigkeit kann bereits einer Ansiedlung von Keimen in der Blase bzw. dem Urogenitaltrakt im Allgemeinen vorgebeugt werden und kann vor allem zu einer normalen Funktion der Blase beigetragen werden. Die Einnahme der Brausezubereitung direkt als Pulver sowie aufgelöst als Getränk wird für beide Grundrezepturen 1 und 2 jeweils dokumentiert und wie nachfolgend in Tabelle 2a und 2b wiedergegeben evaluiert.

Zur direkten Einnahme wird eine Menge des Pulvers, die einer Produkteinheit entspricht (vgl. Tab. 1, d.h. 1.525,0 mg), oral appliziert. Das Pulver wird im Mund gehalten, bis eine Verflüssigung festgestellt werden kann, und dann heruntergeschluckt. Die sensorischen Eigenschaften und Empfindungen sind festgehalten und für die Auswertung gemäß Tabelle 2a bzw. 2b gemittelt worden.

Zur Herstellung eines Getränks aus der Brausezubereitung wird jeweils eine Menge des Pulvers, die einer Produkteinheit entspricht, d.h. 1.525,0 mg, in 500 mL Wasser in einer geschlossenen Flasche aufgelöst.

Auflöseverhalten und Schaumbildung werden optisch dokumentiert. Nach vollständiger und rückstandsfreier Auflösung des Brausepulvers ist das erhaltene Getränk verköstigt worden und sind die sensorischen Eigenschaften und Empfindungen festgehalten und für die Auswertung gemäß Tabelle 2a bzw. 2b gemittelt worden. Es wird zur Evaluation des Löslichkeitsverhaltens dabei sowohl eine Testreihe mit kaltem Wasser als auch mit lauwarmen Wasser durchgeführt.

### b. Brausetablette

Die Brausetablette, wiederum auf Basis je der Grundrezeptur 1 und 2, ist so formuliert, dass diese direkt oral sowie in Wasser aufgelöst als Getränk eingenommen werden kann.

Ebenso wie im Falle des Pulvers, bietet sich die direkte orale Einnahme der Tablette in akuten Fällen, bspw. bei der therapeutischen Behandlung von Infektionen des Urogenitaltraktes, sowie, hinsichtlich der Verwendung als Nahrungsergänzungsmittel, bei einem erhöhten Nährstoffbedarf, bspw. nach sportlicher Belastung oder aufgrund bspw. alterstypischem Nährstoffmangel, an. Als Tablette bietet sich die erfindungsgemäße Darreichungsform außerdem für die unkomplizierte und sichere Versorgung mit Nähr- und Mineralstoffen unterwegs und/oder auch mehrmals täglich an.

Die Einnahme als Getränk bietet sich insbesondere im Rahmen der Verwendung als Nahrungsergänzungsmittel sowie für die prophylaktische Behandlung von Infektionen des Urogenitaltraktes an, indem eine zusätzliche Zufuhr von Flüssigkeit erreicht und so insgesamt zu einer normalen Funktion der Blase beigetragen werden kann. Die Einnahme der Brausezubereitung direkt als Tablette sowie aufgelöst als Getränk wird dokumentiert und wie nachfolgend in Tabelle 2a und 2b wiedergegeben evaluiert.

Zur direkten Einnahme wird eine Tablette, die einer Produkteinheit entspricht (vgl. Tab. 1, d.h. 1.525,0 mg), oral appliziert. Die Tablette wird im Mund gehalten und gelutscht, bis ein nahezu vollständiges Auflösen bzw. Verflüssigen festgestellt werden kann und wird heruntergeschluckt. Die sensorischen Eigenschaften und Empfindungen sind festgehalten und für die Auswertung gemäß Tabelle 2a bzw. 2b gemittelt worden.

Zur Herstellung eines Getränks aus der Brausetablette wird jeweils eine Tablette, die einer Produkteinheit entspricht, d.h. 1.525,0 mg, in 500 mL Wasser in einer geschlossenen Flasche aufgelöst.

Auflöseverhalten und Schaumbildung werden optisch dokumentiert. Nach vollständiger und rückstandsfreier Auflösung der Brausetablette wird das erhaltene Getränk verköstigt und werden die sensorischen Eigenschaften und Empfindungen festgehalten und für die Auswertung gemäß Tabelle 2a bzw. 2b gemittelt. Es wird zur Evaluation des Löslichkeitsverhaltens dabei sowohl eine Testreihe mit kaltem Wasser als auch mit lauwarmen Wasser durchgeführt.

**Tabelle 2a:**

| | | **Grundrezeptur 1** | | | | | |
|---|---|---|---|---|---|---|---|
| **Beurteilungs kriterien** | | ***Brausepulver*** | | | ***Brausetablette*** | | |
| | | Pulver | Getränk | | Tablette | Getränk | |
| | | | *kalt* | *lauwarm* | | *kalt* | *lauwarm* |
| | | | | | | | |
| Löslichkeit quantitativ* | | +++ (im Mund) | +++ (in Wasser) | +++ (in Wasser) | ++ (im Mund) | ++ (in Wasser) | +++ (in Wasser) |
| Löslichkeit qualitativ** | | + (im Mund) | ++ (in Wasser) | +++ (in Wasser) | + (im Mund) | + (in Wasser) | ++ (in Wasser) |
| Geschmack | | ++ | +++ | 0 | ++ | +++ | 0 |
| Optik | | + | +++ | +++ | ++ | +++ | +++ |
| Geruch | | + | ++ | + | + | ++ | + |
| Mundgefühl | | + | +++ | +++ | + | +++ | +++ |
| Schluckgefühl | | + | +++ | +++ | ++ | +++ | +++ |
| Anwendung | | +++ | ++ | ++ | +++ | ++ | ++ |

| | | | | | | | |
|---|---|---|---|---|---|---|---|
| * d.h., ob bzw. inwiefern sich die Darreichungsform vollständig und rückstandsfrei löst* ** d.h. wie schnell bzw. auf welche (bspw. ob auf eine optisch animierende) Weise sich die Darreichungsform löst | | | | | | | |

### Legende:

| sehr gut | gut | befriedigend | ausreichend | mangelhaft | ungenügend |
|---|---|---|---|---|---|
| +++ | ++ | + | 0 | - | -- |

**Tabelle 2b:**

| | | **Grundrezeptur 2** | | | | | |
|---|---|---|---|---|---|---|---|
| **Beurteilungs kriterien** | | ***Brausepulver*** | | | ***Brausetablette*** | | |
| | | Pulver | Getränk | | Tablette | Getränk | |
| | | | *kalt* | *lauwarm* | | *kalt* | *lauwarm* |
| | | | | | | | |
| Löslichkeit quantitativ* | | +++ (im Mund) | +++ (in Wasser) | +++ (in Wasser) | +++ (im Mund) | +++ (in Wasser) | +++ (in Wasser) |
| Löslichkeit qualitativ** | | +++ (im Mund) | +++ (in Wasser) | +++ (in Wasser) | ++ (im Mund) | +++ (in Wasser) | +++ (in Wasser) |
| Geschmack | | ++ | +++ | + | ++ | +++ | + |
| Optik | | + | +++ | +++ | ++ | +++ | +++ |
| Geruch | | + | ++ | + | + | ++ | + |
| Mundgefühl | | ++ | +++ | +++ | ++ | +++ | +++ |
| Schluckgefühl | | + | +++ | +++ | ++ | +++ | +++ |
| Anwendung | | +++ | +++ | ++ | +++ | +++ | ++ |

| | | | | | | | |
|---|---|---|---|---|---|---|---|
| * d.h., ob bzw. inwiefern sich die Darreichungsform vollständig und rückstandsfrei löst* ** d.h. wie schnell bzw. auf welche (bspw. ob auf eine optisch animierende) Weise sich die Darreichungsform löst | | | | | | | |

### Legende:

| sehr gut | gut | befriedigend | ausreichend | mangelhaft | ungenügend |
|---|---|---|---|---|---|
| +++ | ++ | + | 0 | - | -- |

Das Brausepulver zeichnet sich insgesamt durch ein hervorragendes Auflöseverhalten aus. Eine Produkt- bzw. Dosiereinheit des Brausepulvers löst sich in wenigen Minuten vollständig und rückstandsfrei sowohl in wässriger Lösung als auch bei direkter oraler Applikation.

Die Brausetablette zeichnet sich ebenfalls durch ein insgesamt hervorragendes sowie auch charakteristisches Auflöseverhalten aus. Eine Produkt- bzw. Dosiereinheit der erfindungsgemäßen Darreichungsform benötigt ca. 3 bis 4,5 min bis zur vollständigen und rückstandsfreien Auflösung in Wasser. Insbesondere wenn zusätzliche Sprengmittel bzw. Zerfallsbeschleuniger wie etwa vernetzte Natriumcarboxymethylcellulose (Croscarmellose, vgl. Rezeptur 2) in der Darreichungsform eingesetzt werden, kann die Auflösezeit auf unter 3,5 min und sogar auf ca. 3 min verringert werden. Diese kurze Dauer bis zur vollständigen und rückstandsfreien Auflösung der Darreichungsform wird durch eine Optimierung der Härte und der Zusammensetzung erreicht.

Die Schaumbildung ist, ebenso wie im Falle des Pulvers, vergleichsweise gering, insbesondere auch wenn die Darreichungsform in einem geschlossenen Gefäß, bspw. einer Flasche, mit genügend Luftraum aufgelöst wird.

Auf Grund der nahezu kugelförmigen Ausgestaltung der Brausetablette, insbesondere in Form einer Kugel wie in den Figuren 1, 2A und 2B exemplarisch wiedergegeben, zeigt die Brausetablette ein charakteristisches Auflöseverhalten, bei dem die Tablette auf der Wasseroberfläche aufschwimmt und - bedingt durch den Auflösevorgang im Zusammenspiel mit dem Aufsprudeln des Brausesatzes - eine als tanzend erscheinende Taumelbewegung ausführt. Dieses Auflöseverhalten ist einerseits vorteilhaft, um das vollständige und rückstandsfreie Auflösen der Tablette gut nachverfolgen zu können, und andererseits optisch ansprechend, so dass eine erhöhte Motivation und Wahrscheinlichkeit für die Anwendung der Darreichungsform als Getränk erzielt wird, so dass letztlich leichter zu einer zusätzlichen Flüssigkeitsaufnahme animiert werden kann bzw. wird. Es wird am Ende des Auflösevorgangs, ebenso wie im Falle des Pulvers, ein leicht sprudelndes bzw. brausiges, kohlensäurehaltiges Getränk erhalten.

Die zusätzliche Flüssigkeitsaufnahme bei Einnahme der Darreichungsform als Getränk ist sowohl für die Verwendung als Nahrungsergänzungsmittel als auch für die therapeutische Behandlung von Infektionen des Urogenitaltraktes vorteilhaft, indem so eine höhere Durchspülung der Blase erreicht wird, die im Zusammenhang mit Prävention ebenso wie Prophylaxe bzw. Therapie insgesamt vorteilhaft bzw. förderlich für die Blasengesundheit ist. Die erfindungsgemäßen Darreichungsformen zeichnen sich insgesamt durch als durchweg positiv zu bewertende Anwendungseigenschaften aus.

Für die direkte Applikation als Pulver bzw. Tablette wird eine gute bis sehr Löslichkeit der Zusammensetzung im Mund festgestellt. Das Brausepulver löst sich sehr gut, vollständig sowie zügig im Mund auf, wobei eine angenehm sprudelnde Empfindung festgestellt werden kann.

Die Tablette löst sich gegenüber dem Pulver etwas langsamer und ist wenige Minuten zu lutschen, bis eine nahezu vollständige bzw. vollständige Auflösung erreicht werden kann. Es wird währenddessen eine angenehme sprudelnde Empfindung und eine insgesamt gute Sensorik beschrieben.

Pulver wie auch Tablette zeichnen sich durch einen fruchtigen und als insgesamt angenehmen, partiell etwas spritzig-säuerlich wiedergegeben, belebenden Geschmack aus. Der Geruch wird ebenfalls als angenehm beschrieben. Besonders die einfache Handhabung von Pulver und Tablette bei der Direktanwendung wird als vorteilhaft hervorgehoben.

Verwendet als Getränk zeichnet sich die erfindungsgemäße Darreichungsform sowohl als Pulver als auch als Tablette durch eine insgesamt sehr gute Löslichkeit aus, insbesondere wobei sich die Darreichungsform rückstandsfrei löst. Besonders gut löst sich die Darreichungsform dabei in lauwarmen Wasser, während in kaltem Wasser ein etwas langsameres Lösen beobachtet wird, wobei stets Auflösezeiten für die Dosiereinheit von deutlich unter 5 min und idealerweise um 3 min eingehalten werden.

Als gekühltes Getränk zeichnet sich die Darreichungsform durch einen sehr guten Geschmack aus. Geruch, Mund- und Schluckgefühl werden für das Getränk als sehr gut bewertet und die Anwendung als unkompliziert ausgelobt.

Die ansprechende optische Erscheinung insbesondere der Brausetablette wie auch der Getränkezubereitung, die eine als appetitlich bewertete rosa bzw. pinke Färbung zeigt, ist außerdem positiv bewertet worden.

Insgesamt eignet sich die erfindungsgemäße Darreichungsform also ideal sowohl für die Anwendung als Pulver als auch als Tablette, wobei sowohl eine direkte orale Applikation als auch die Einnahme als Getränk, in dem die Darreichungsform aufgelöst wird, mit sehr guten Anwendungseigenschaften einher geht.

### 3. Untersuchung der Wirksamkeit der erfindungsgemäßen Darreichungsform als Brausezubereitung

10 Probandinnen im Alter zwischen 48 und 79 Jahren mit diversen Vorerkrankungen (Diabetes, Hypertonie, Adipositas etc.), welche jeweils unter rezidivierender Zystitis leiden, erhalten in der nicht-akuten bzw. nicht-entzündlichen Phase über einen Zeitraum von 8 Wochen zweimal täglich eine Dosiereinheit der erfindungsgemäßen Darreichungsform, jeweils verabreicht aufgelöst in 250 mL Trinkwasser als Getränkezubereitung. Zu Beginn des Verabreichungsintervalls geben 8 von 10 Probandinnen an, seit 2 Wochen oder länger Infekt-frei in Bezug auf eine akute Zystitis zu sein. Bei 2 der 10 Probandinnen liegt eine Zystitis weniger als 2 Wochen zurück.

Sechs von 10 Probandinnen berichten von einer deutlichen Verbesserung gegenüber des vor der Therapie liegenden Komforts und der vor der Therapie liegenden Dringlichkeit beim Wasserlassen. Die Probandinnen empfinden keine Anzeichen einer wiederkehrenden Zystitis.

Zwei von 10 Probandinnen können keine deutliche Verbesserung des Komforts beim Wasserlassen feststellen, geben jedoch an, eine Milderung gegenüber der vor der Therapie liegenden Dringlichkeit zu empfinden. Die Probandinnen empfinden außerdem keine Anzeichen einer wiederkehrenden Zystitis.

Eine von 10 Probandinnen berichtet von einer deutlichen Verbesserung gegenüber des vor der Therapie liegenden Komforts und der vor der Therapie liegenden Dringlichkeit beim Wasserlassen, und beschreibt zudem, eine mild abführende Wirkung der Darreichungsform wahrgenommen zu haben. Anzeichen einer wiederkehrenden Zystitis werden von dieser Probandin nicht empfunden.

Eine von 10 Probandinnen erkrankt im Verlauf der achtwöchigen Verabreichungsdauer an einer Zystitis. Es wird aber ein gegenüber einer vor der Therapie liegenden Infektion milderer Verlauf beschrieben sowie eine Verkürzung der akuten Phase der Zystitis.

In einem weiteren Versuch erhalten 5 weitere Probandinnen im Alter zwischen 52 und 78 Jahren mit diversen Vorerkrankungen (Hypertonie, Adipositas etc.), welche jeweils unter rezidivierender Zystitis leiden, über einen Zeitraum von 8 Wochen dreimal täglich eine Dosiereinheit der erfindungsgemäßen Darreichungsform, jeweils verabreicht aufgelöst in 250 mL Trinkwasser als Getränkezubereitung.

Zu Beginn des Verabreichungsintervalls geben 4 von 5 Probandinnen an, seit 2 Wochen oder länger Infekt-frei in Bezug auf eine akute Zystitis zu sein. Bei einer der 10 Probandinnen liegt eine Zystitis weniger als 2 Wochen zurück.

Alle 5 Probandinnen berichten von einer deutlichen Verbesserung gegenüber des vor der Therapie liegenden Komforts und der vor der Therapie liegenden Dringlichkeit beim Wasserlassen. Die Probandinnen empfinden keine Anzeichen einer wiederkehrenden Zystitis.

Drei Probandinnen geben zusätzlich an, sich insgesamt vitaler, besser mit Nährstoffen versorgt und körperlich ausbalancierter zu fühlen.

Die Untersuchungen zur Wirksamkeit der erfindungsgemäßen Darreichungsform bzw. zu einem spürbaren ernährungsphysiologischen Effekt für die Verwendung dieser Darreichungsform als Nahrungsergänzungsmittel zeigen also ein ganz überwiegend sehr positives Wirksamkeitsverhalten der erfindungsgemäßen Darreichungsform, indem insbesondere eine Steigerung bzw. Verbesserung des Komforts beim Wasserlassen angegeben wird sowie auch eine subjektive Verbesserung der Allgemeinsituation in Bezug auf die Blasengesundheit der Probandinnen wie auch deren allgemeine physiologische Konstitution. Die erfindungsgemäße Darreichungsform gestattet es demnach also vorteilhaft, auf ansprechende Weise zum Trinken zu animieren und so den Harnfluss mild anzutreiben sowie die Blase auf eine ernährungsphysiologisch wertvolle wie auch wirksame Weise vor Bakterien zu schützen und gesund zu erhalten.

Zusätzlich werden der Elektrolythaushalt, die Eisenaufnahme und das Immunsystem gefördert, was sich subjektiv in einer allgemein als gesteigert empfundenen Vitalität und Fitness ausdrückt. Weiterhin trägt die erfindungsgemäße Darreichungsform auch effektiv zu einer Prophylaxe von Harnwegsinfektionen bei und leistet auch auf diese Weise eine wirksamen Beitrag zur Förderung und Aufrechterhaltung der Blasengesundheit.

### Bezugszeichenliste:

| | | | |
|---|---|---|---|
| 1 | Darreichungsform | 2b | Halbkugel |
| 2a | Halbkugel | 3 | mittig umlaufender Steg |

## Patentansprüche

1. Darreichungsform, insbesondere Nahrungsergänzungsmittel- und/oder pharmazeutische Darreichungsform, insbesondere für die orale Applikation, vorzugsweise zur Verwendung bei der prophylaktischen und/oder therapeutischen Behandlung von Infektionen und/oder entzündlichen Erkrankungen des Urogenitaltraktes, insbesondere der Harnwege, und/oder zur unterstützenden präventiven und/oder kurativen Sicherstellung und/oder Förderung der Blasengesundheit,
wobei die Darreichungsform als Brausezubereitung, insbesondere in Form eines losen oder gepressten Pulvers, vorzugsweise in Form einer Brausetablette, ausgebildet ist,
wobei die Darreichungsform eine Kombination von mindestens einer Proanthocyanidine-enthaltenden Wirkstoffkomponente einerseits und mindestens einer diuretisch-wirksamen und/oder entzündungshemmenden Wirkstoffkomponente andererseits umfasst, und
wobei die Kombination
a) mindestens einen Extrakt, insbesondere mindestens zwei Extrakte, von Pflanzen aus der Familie der Heidekrautgewächse (Ericaceae), insbesondere der Gattung Heidelbeere (Vaccinium), einerseits und
b) mindestens einen Extrakt von Pflanzen aus der Familie der Brennnesselgewächse (Urticaceae) andererseits
enthält.

2. Darreichungsform, insbesondere Nahrungsergänzungsmittel- und/oder pharmazeutische Darreichungsform, insbesondere für die orale Applikation, vorzugsweise zur Verwendung bei der prophylaktischen und/oder therapeutischen Behandlung von Infektionen und/oder entzündlichen Erkrankungen des Urogenitaltraktes, insbesondere der Harnwege, und/oder zur unterstützenden präventiven und/oder kurativen Sicherstellung und/oder Förderung der Blasengesundheit, insbesondere Darreichungsform nach Anspruch 1,
wobei die Darreichungsform in Kombination und in jeweils wirksamen, insbesondere in jeweils Nahrungsergänzungsmittel- und/oder pharmazeutisch wirksamen, Mengen enthält:
a) mindestens eine Proanthocyanidine-enthaltende Wirkstoffkomponente in Form mindestens eines Extrakts, insbesondere mindestens zweier Extrakte, von Pflanzen aus der Familie der Heidekrautgewächse, insbesondere der Gattung Heidelbeere (Vaccinium), (Ericaceae) und
b) mindestens eine diuretisch-wirksame und/oder entzündungshemmende Wirkstoffkomponente in Form mindestens eines Extrakts von Pflanzen aus der Familie der Brennnesselgewächse (Urticaceae).

3. Darreichungsform nach Anspruch 1 oder Anspruch 2,
wobei a) der mindestens eine Extrakt, insbesondere die mindestens zwei Extrakte, von Pflanzen aus der Familie der Heidekrautgewächse (Ericaceae), , insbesondere der Gattung Heidelbeere (Vaccinium), ausgewählt ist/sind aus der Gruppe von Großfrüchtiger Moosbeere (*Vaccinium macrocarpon Aiton*), Kleinfrüchtiger Moosbeere (*Vaccinium microcarpum*), Gewöhnlicher Moosbeere (*Vaccinium oxycoccos* L.), Preiselbeere *(Vaccinium vitis-idaea* L.), Blaubeere (*Vaccinium myrtillus* L.), Amerikanischer Heidelbeere *(Vaccinium corymbosum* L.), Kanadischer Blaubeere (*Vaccinium myrtilloides* MICHX.) und/oder deren Mischungen, insbesondere Großfrüchtiger Moosbeere (*Vaccinium macrocarpon Aiton*), Preiselbeere *(Vaccinium vitis-idaea* L.) und/oder deren Mischungen;
insbesondere wobei die Darreichungsform in Kombination und in jeweils wirksamen, insbesondere in jeweils Nahrungsergänzungsmittel- und/oder pharmazeutisch wirksamen, Mengen mindestens zwei, insbesondere genau zwei, Extrakte aus Pflanzen der Familie der Heidekrautgewächse (Ericaceae), insbesondere der Gattung Heidelbeere (Vaccinium), enthält; vorzugsweise wobei a) einen ersten Extrakt a1) mindestens einen Extrakt von Preiselbeere (*Vaccinium vitis-idaea* L.) und einen zweiten Extrakt a2) mindestens einen Extrakt von Großfrüchtiger Moosbeere (*Vaccinium macrocarpon Aiton*) enthält, insbesondere hieraus besteht; und/oder
wobei b) der mindestens eine Extrakt von Pflanzen aus der Familie der Brennnesselgewächse (Urticaceae) ausgewählt ist aus der Gruppe von Großer Brennnessel (*Urtica dioica*), Kleiner Brennnessel (*Urtica urens*) und/oder deren Mischung;
insbesondere wobei b) einen Extrakt b1) mindestens einen Extrakt von Brennnesselblättern (*Urticae folium*) enthält, insbesondere hieraus besteht.

4. Darreichungsform nach einem der vorangehenden Ansprüche,
wobei die Darreichungsform in Kombination und in jeweils wirksamen, insbesondere in jeweils Nahrungsergänzungsmittel- und/oder pharmazeutisch wirksamen, Mengen enthält:
a1) mindestens einen Extrakt von Preiselbeere (*Vaccinium vitis-idaea* L.),
a2) mindestens einen Extrakt von Großfrüchtiger Moosbeere (*Vaccinium macrocarpon Aiton)* und
b1) mindestens einen Extrakt von Brennnesselblättern (*Urticae folium*);
und/oder
wobei die Darreichungsform als Wirkstoffkomponente a) eine Kombination von a1) mindestens einem Extrakt von Preiselbeere (*Vaccinium vitis-idaea* L.) und
a2) mindestens einem Extrakt von Großfrüchtiger Moosbeere (*Vaccinium macrocarpon Aiton*) und als Wirkstoffkomponente b) b1) mindestens einen Extrakt von Brennnesselblättern (*Urticae folium*) enthält.

5. Darreichungsform, insbesondere Nahrungsergänzungsmittel- und/oder pharmazeutische Darreichungsform, insbesondere für die orale Applikation, vorzugsweise zur Verwendung bei der prophylaktischen und/oder therapeutischen Behandlung von Infektionen und/oder entzündlichen Erkrankungen des Urogenitaltraktes, insbesondere der Harnwege, und/oder zur unterstützenden präventiven und/oder kurativen Sicherstellung und/oder Förderung der Blasengesundheit, insbesondere Darreichungsform nach einem der Ansprüche 1 bis 4, wobei die Darreichungsform in Kombination und in jeweils wirksamen, insbesondere in jeweils Nahrungsergänzungsmittel- und/oder pharmazeutisch wirksamen, Mengen enthält:
a1) mindestens einen Extrakt von Preiselbeere *(Vaccinium vitis-idaea* L.),
a2) mindestens einen Extrakt von Großfrüchtiger Moosbeere *(Vaccinium macrocarpon Aiton)* und
b1) mindestens einen Extrakt von Brennnesselblättern *(Urticae folium).*

6. Darreichungsform nach einem der vorangehenden Ansprüche,
wobei die Extrakte jeweils zusammen mit und/oder in Kombination mit einem Träger formuliert sind und/oder vorliegen;
insbesondere wobei der Träger ein wasserlöslicher oder wasserdispergierbarer, insbesondere Kohlenhydrat-basierter und/oder -haltiger, vorzugsweise Oligo- und/oder Polysaccharid-basierter und/oder -haltiger, Träger ist, bevorzugt Maltodextrin ist, und/oder
insbesondere wobei die Extrakte den Träger jeweils in Mengen in einem Bereich von 2 bis 70 Gew.-%, insbesondere 3 bis 60 Gew.-%, vorzugsweise 4 bis 55 Gew.-%, bevorzugt 5 bis 50 Gew.-%, bezogen auf die Gesamtzusammensetzung des Extrakts, enthalten.

7. Darreichungsform nach einem der vorangehenden Ansprüche,
wobei a1) der mindestens eine Extrakt von Preiselbeere (*Vaccinium vitis-idaea* L.) jeweils zusammen mit und/oder in Kombination mit einem Träger formuliert ist und/oder vorliegt und/oder wobei a2) der mindestens eine Extrakt von Großfrüchtiger Moosbeere (*Vaccinium macrocarpon Aiton*) jeweils zusammen mit und/oder in Kombination mit einem Träger formuliert ist und/oder vorliegt und/oder wobei b1) der mindestens eine Extrakt von Brennnesselblättern (*Urticae folium*) jeweils zusammen mit und/oder in Kombination mit einem Träger formuliert ist und/oder vorliegt;
insbesondere wobei der Träger ein wasserlöslicher oder wasserdispergierbarer, insbesondere Kohlenhydrat-basierter und/oder -haltiger, vorzugsweise Oligo- und/oder Polysaccharid-basierter und/oder -haltiger, Träger ist, bevorzugt Maltodextrin ist, und/oder
insbesondere wobei der oder die Extrakte den Träger jeweils in Mengen in einem Bereich von 2 bis 70 Gew.-%, insbesondere 3 bis 60 Gew.-%, vorzugsweise 4 bis 55 Gew.-%, bevorzugt 5 bis 50 Gew.-%, bezogen auf die Gesamtzusammensetzung des Extrakts, enthält/enthalten.

8. Darreichungsform nach Anspruch 7,
wobei a1) der Extrakt von Preiselbeere (*Vaccinium vitis-idaea* L.) den Träger in Mengen in einem Bereich von 2 bis 10 Gew.-%, insbesondere 3 bis 8 Gew.-%, vorzugsweise 4 bis 6 Gew.-%, bevorzugt 5 Gew.-%, bezogen auf die Gesamtzusammensetzung des Extrakts, enthält und/oder
wobei a2) der Extrakt von Großfrüchtiger Moosbeere (*Vaccinium macrocarpon Aiton*) den Träger in Mengen in einem Bereich von 20 bis 70 Gew.-%, insbesondere 25 bis 60 Gew.-%, vorzugsweise 30 bis 55 Gew.-%, bevorzugt 30 bis 50 Gew.-%, bezogen auf die Gesamtzusammensetzung des Extrakts, enthält und/oder
wobei b1) der mindestens eine Extrakt von Brennnesselblättern (*Urticae folium*), den Träger in Mengen in einem Bereich von 20 bis 70 Gew.-%, insbesondere 25 bis 60 Gew.-%, vorzugsweise 30 bis 55 Gew.-%, bevorzugt 30 bis 50 Gew.-%, bezogen auf die Gesamtzusammensetzung des Extrakts, enthält.

9. Darreichungsform nach einem der vorangehenden Ansprüche,
wobei die Extrakte jeweils als Trockenextrakte ausgebildet sind; und/oder
wobei die Extrakte in der Darreichungsform jeweils in Mengen in einem Bereich von 5 bis 45 Gew.-%, insbesondere 10 bis 35 Gew.-%, vorzugsweise 15 bis 29 Gew.-%, bevorzugt 17 bis 25 Gew.-%, bezogen auf die Darreichungsform und berechnet auf Basis der Gesamtzusammensetzung der Extrakte, enthalten sind.

10. Darreichungsform nach einem der vorangehenden Ansprüche,
wobei a1) der Extrakt von Preiselbeere (*Vaccinium vitis-idaea* L.) in der Darreichungsform in Mengen in einem Bereich von 1 bis 20 Gew.-%, insbesondere 3 bis 15 Gew.-%, vorzugsweise 4 bis 12 Gew.-%, bevorzugt 5 bis 10 Gew.-%, bezogen auf die Darreichungsform und berechnet auf Basis der Gesamtzusammensetzung des Extrakts, enthalten ist und/oder
wobei a2) der Extrakt von Großfrüchtiger Moosbeere (*Vaccinium macrocarpon Aiton*) in der Darreichungsform in Mengen in einem Bereich von 1 bis 20 Gew.-%, insbesondere 3 bis 15 Gew.-%, vorzugsweise 4 bis 12 Gew.-%, bevorzugt 5 bis 10 Gew.-%, bezogen auf die Darreichungsform und berechnet auf Basis der Gesamtzusammensetzung des Extrakts, enthalten ist und/oder
wobei b1) der Extrakt von Brennnesselblättern *(Urticae folium)* in der Darreichungsform in Mengen in einem Bereich von 1 bis 20 Gew.-%, insbesondere 3 bis 15 Gew.-%, vorzugsweise 4 bis 12 Gew.-%, bevorzugt 5 bis 10 Gew.-%, bezogen auf die Darreichungsform und berechnet auf Basis der Gesamtzusammensetzung des Extrakts, enthalten ist.

11. Darreichungsform nach einem der vorangehenden Ansprüche,
wobei die Extrakte in der Darreichungsform in Mengen in einem Bereich von 2 bis 30 Gew.-%, insbesondere 4 bis 22 Gew.-%, vorzugsweise 6 bis 19 Gew.-%, bevorzugt 7 bis 17 Gew.-%, bezogen auf die Darreichungsform und berechnet auf Basis der Extraktzusammensetzung ohne Träger und/oder auf Basis der eingesetzten Trockenextraktmenge, enthalten sind; und/oder
wobei die Extrakte ein Droge/Extrakt-Verhältnis (DEV) in einem Bereich von 75 : 1 bis 1 : 10, insbesondere 65 : 1 bis 1 : 5, vorzugsweise 60 : 1 bis 1 : 1, bevorzugt 55 : 1 bis 3 : 1, jeweils bezogen auf die Gesamtzusammensetzung der Extrakte, aufweisen; und/oder
wobei die Darreichungsform einen Proanthocyanidin-Gehalt in einem Bereich von 0,01 bis 5 Gew.-%, insbesondere 0,05 bis 3,5 Gew.-%, vorzugsweise 0,1 bis 3 Gew.-%, bevorzugt 0,5 bis 2,5 Gew.-%, bezogen auf die Darreichungsform, aufweist.

12. Darreichungsform nach einem der vorangehenden Ansprüche,
wobei a) der mindestens einen Extrakt, insbesondere die mindestens zwei Extrakte, von Pflanzen aus der Familie der Heidekrautgewächse (Ericaceae) , insbesondere der Gattung Heidelbeere (Vaccinium), vorzugsweise a1) der mindestens eine Extrakt von Preiselbeere (*Vaccinium vitis-idaea*), bevorzugt die Darreichungsform, einen Proanthocyanidin-Gehalt in einem Bereich von 0,01 bis 5 Gew.-%, insbesondere 0,05 bis 3,5 Gew.-%, vorzugsweise 0,1 bis 3 Gew.-%, bevorzugt 0,5 bis 2,5 Gew.-%, bezogen auf die Gesamtzusammensetzung des Extrakts, vorzugsweise bezogen auf die Darreichungsform, aufweist/aufweisen;
insbesondere wobei der Proanthocyanidin-Gehalt der Darreichungsform zumindest im Wesentlichen dem Proanthocyanidin-Gehalt a) des mindestens einen Extrakts, insbesondere der mindestens zwei Extrakte, von Pflanzen aus der Familie der Heidekrautgewächse (Ericaceae), insbesondere der Gattung Heidelbeere (Vaccinium), vorzugsweise a1) des mindestens einen Extrakts von Preiselbeere (*Vaccinium vitis-idaea*), entspricht; und/oder
wobei die diuretisch-wirksame und/oder entzündungshemmende Wirkstoffkomponente, insbesondere b), vorzugsweise b1), Kaffeoyläpfelsäure und/oder Chlorogensäure als Wirkstoffe enthält.

13. Darreichungsform nach einem der vorangehenden Ansprüche,
wobei die Darreichungsform ein antibakteriell-wirksames Saccharid, insbesondere Monosaccharid, vorzugsweise Mannose, besonders bevorzugt D-Mannose, umfasst;
insbesondere wobei die Darreichungsform das Saccharid in Mengen in einem Bereich von 0,0001 bis 10 Gew.-%, insbesondere 0,001 bis 7,5 Gew.-%, vorzugsweise 0,01 bis 5 Gew.-%, bevorzugt 0,1 bis 3 Gew.-%, bezogen auf die Darreichungsform, enthält.

14. Darreichungsform, insbesondere Nahrungsergänzungsmittel- und/oder pharmazeutische Darreichungsform, insbesondere für die orale Applikation, vorzugsweise zur Verwendung bei der prophylaktischen und/oder therapeutischen Behandlung von Infektionen und/oder entzündlichen Erkrankungen des Urogenitaltraktes, insbesondere der Harnwege, und/oder zur unterstützenden präventiven und/oder kurativen Sicherstellung und/oder Förderung der Blasengesundheit, insbesondere Darreichungsform nach einem der vorangehenden Ansprüche,
wobei die Darreichungsform in Kombination und in jeweils wirksamen, insbesondere in jeweils Nahrungsergänzungsmittel- und/oder pharmazeutisch wirksamen, Mengen enthält:
a1) mindestens einen Extrakt von Preiselbeere *(Vaccinium vitis-idaea* L.),
a2) mindestens einen Extrakt von Großfrüchtiger Moosbeere *(Vaccinium macrocarpon Aiton)* und
b1) mindestens einen Extrakt von Brennnesselblättern *(Urticae folium).*
wobei die Darreichungsform als Brausezubereitung, insbesondere in Form eines losen oder gepressten Pulvers, vorzugsweise in Form einer Brausetablette, ausgebildet ist und
wobei die Extrakte jeweils zusammen mit und/oder in Kombination mit einem Träger formuliert sind und/oder vorliegen, insbesondere wobei der Träger ein wasserlöslicher oder wasserdispergierbarer, insbesondere Kohlenhydrat-basierter und/oder -haltiger, vorzugsweise Oligo- und/oder Polysaccharid-basierter und/oder -haltiger, Träger ist, bevorzugt Maltodextrin ist;
wobei a1) der Extrakt von Preiselbeere *(Vaccinium vitis-idaea* L.) den Träger in Mengen in einem Bereich von 2 bis 10 Gew.-%, bezogen auf die Gesamtzusammensetzung des Extrakts, enthält und
wobei a2) der Extrakt von Großfrüchtiger Moosbeere (*Vaccinium macrocarpon Aiton*) den Träger in Mengen in einem Bereich von 20 bis 70 Gew.-%, bezogen auf die Gesamtzusammensetzung des Extrakts, enthält und
wobei b1) der mindestens eine Extrakt von Brennnesselblättern (*Urticae folium*), den Träger in Mengen in einem Bereich von 20 bis 70 Gew.-%, bezogen auf die Gesamtzusammensetzung des Extrakts, enthält, und
insbesondere wobei a1) der Extrakt von Preiselbeere *(Vaccinium vitis-idaea* L.) in der Darreichungsform in Mengen in einem Bereich von 1 bis 20 Gew.-%, bezogen auf die Darreichungsform und berechnet auf Basis der Gesamtzusammensetzung des Extrakts, enthalten ist und ein Droge/Extrakt-Verhältnis (DEV) in einem Bereich von 75:1 bis 35:1, bezogen auf die Gesamtzusammensetzung des Extrakts, aufweist und
insbesondere wobei a2) der Extrakt von Großfrüchtiger Moosbeere (*Vaccinium macrocarpon Aiton*) in der Darreichungsform in Mengen in einem Bereich von 1 bis 20 Gew.-%, bezogen auf die Darreichungsform und berechnet auf Basis der Gesamtzusammensetzung des Extrakts, enthalten ist und ein Droge/Extrakt-Verhältnis (DEV) in einem Bereich von 20 : 1 bis 1 : 10, bezogen auf die Gesamtzusammensetzung des Extrakts, aufweist und
insbesondere wobei b1) der Extrakt von Brennnesselblättern *(Urticae folium)* in der Darreichungsform in Mengen in einem Bereich von 1 bis 20 Gew.-%, bezogen auf die Darreichungsform und berechnet auf Basis der Gesamtzusammensetzung des Extrakts, enthalten ist und ein Droge/Extrakt-Verhältnis (DEV) in einem Bereich von 20: 1 bis 1:10, bezogen auf die Gesamtzusammensetzung des Extrakts, aufweist, und
wobei die Darreichungsform, einen Proanthocyanidin-Gehalt in einem Bereich von 0,01 bis 5 Gew.-%, insbesondere 0,05 bis 3,5 Gew.-%, vorzugsweise 0,1 bis 3 Gew.-%, bevorzugt 0,5 bis 2,5 Gew.-%, bezogen auf die Darreichungsform, aufweist.

15. Darreichungsform nach einen der vorangehenden Ansprüche zur Verwendung bei der prophylaktischen und/oder therapeutischen Behandlung von Infektionen und/oder entzündlichen Erkrankungen des Urogenitaltraktes, insbesondere der Harnwege, und/oder zur prophylaktischen oder therapeutischen Behandlung von insbesondere chronisch rezidivierenden Harnwegsinfekten und/oder bei der, insbesondere ergänzenden und/oder unterstützenden, präventiven und/oder kurativen Versorgung mit Nährstoffen und aktiven Inhaltsstoffen zur Sicherstellung und/oder Förderung der Blasengesundheit.

16. Verwendung einer Darreichungsform nach einem der Ansprüche 1 bis 14 im Nahrungsergänzungsbereich, insbesondere zur nicht-therapeutischen und/oder nicht-medizinischen Vorbeugung von Infektionen und/oder entzündlichen Erkrankungen des Urogenitaltraktes, insbesondere der Harnwege, und/oder bei der, insbesondere ergänzenden, präventiven und/oder kurativen Versorgung mit Nährstoffen und aktiven Inhaltsstoffen zur Sicherstellung und/oder Förderung der Blasengesundheit.

17. Brausezubereitung, insbesondere für die orale Applikation, insbesondere in Form eines losen oder gepressten Pulvers, vorzugsweise Brausetablette, bestehend aus einer Darreichungsform nach einem der Ansprüche 1 bis 14, insbesondere zur Verwendung bei der prophylaktischen und/oder therapeutischen Behandlung von Infektionen und/oder entzündlichen Erkrankungen des Urogenitaltraktes, insbesondere der Harnwege, und/oder zur prophylaktischen oder therapeutischen Behandlung von insbesondere chronisch rezidivierenden Harnwegsinfekten und/oder bei der, insbesondere ergänzenden, präventiven und/oder kurativen Versorgung mit Nährstoffen und aktiven Inhaltsstoffen zur Sicherstellung und/oder Förderung der Blasengesundheit.

18. Verwendung einer Brausezubereitung nach Anspruch 17 im Nahrungsergänzungsbereich, insbesondere zur nicht-therapeutischen und/oder nicht-medizinischen Vorbeugung von Infektionen und/oder entzündlichen Erkrankungen des Urogenitaltraktes, insbesondere der Harnwege, und/oder bei der, insbesondere ergänzenden, präventiven und/oder kurativen Versorgung mit Nährstoffen und aktiven Inhaltsstoffen zur Sicherstellung und/oder Förderung der Blasengesundheit.

19. Brausetablette (1), insbesondere umfassend eine Nahrungsergänzungsmittel- und/oder pharmazeutische Darreichungsform, insbesondere für die orale Applikation, vorzugsweise zur Verwendung bei der prophylaktischen und/oder therapeutischen Behandlung von Infektionen und/oder entzündlichen Erkrankungen des Urogenitaltraktes, insbesondere der Harnwege, und/oder zur unterstützenden präventiven und/oder kurativen Sicherstellung und/oder Förderung der Blasengesundheit,
wobei die Brausetablette zumindest im Wesentlichen in Form einer Kugel, insbesondere in Form einer insbesondere oval verzerrten Kugel oder eines Ellipsoids, mit mittig umlaufendem Steg (3), ausgebildet ist;
wobei die Brausetablette eine Darreichungsform nach einem der Ansprüche 1 bis 14 und/oder eine Brausezubereitung nach Anspruch 17 enthält.

20. Verpackungseinheit, enthaltend mindestens eine Darreichungsform, vorzugsweise eine Vielzahl von Darreichungsformen, nach einem der Ansprüche 1 bis 14 und/oder enthaltend mindestens eine Brausezubereitung, vorzugsweise eine Vielzahl von Brausezubereitungen, nach Anspruch 17 und/oder enthaltend mindestens eine Brausetablette, vorzugsweise eine Vielzahl von Brausetabletten, nach Anspruch 19.
